# EUROPEAN PATENT SPECIFICATION

(11) **EP 4 243 883 B1**
(45) Date of publication and mention of the grant of the patent: **18.03.2026**
(21) Application number: 21815601.6
(22) Date of filing: 12.11.2021
(51) Int. Cl.: A61L 2/07, B05B 1/00, B05B 1/34, A61L 2/24, B05B 1/12, B08B 3/00, F26B 3/04, F26B 9/00, F26B 21/10

(54) **STERILISING APPARATUS**
STERILISATIONSVORRICHTUNG
APPAREIL DE STÉRILISATION

(30) Priority: 13.11.2020 GB 202017912
(43) Date of publication of application: 20.09.2023
(73) Proprietor: Strix Limited, Ronaldsway, Isle of Man IM9 2RG (GB)
(72) Inventor: HEWINS, Jacob Alexander John, Santon Isle of Man IM4 1EJ (GB); MORRIS, Leo Stephen, Douglas Isle of Man IM1 3NA (GB)
(74) Representative: Dehns
(86) International application number: PCT/GB2021/052933
(87) International publication number: WO 2022/101638

(56) References cited:
- EP-A1- 3 409 298
- WO-A1-2020/114397
- CN-A- 108 434 497
- CN-A- 109 203 156
- CN-U- 206 745 641
- KR-B1- 101 521 509
- KR-B1- 101 521 509
- RU-C1- 2 335 713

## Description

The present invention is directed towards a sterilising apparatus, particularly an apparatus for sterilising baby bottles and their associated components.

Until a baby has reached around one year old, it is regarded as necessary to sterilise all feeding equipment before each use. For example, feeding bottles, along with their associated parts including teats and covers, must be sterile before use. Sterilisation prior to each use ensures that any microbes, e.g. bacteria, present on the equipment is killed and therefore helps to avoid the baby becoming ill from use of the equipment.

There are various ways of sterilising feeding equipment including: electric steam sterilisers, microwave steam sterilisers, water boilers and cold water sterilisation. As will be appreciated, as feeding equipment is frequently used, there is a corresponding need to frequently sterilise such equipment. Accordingly, a simple, easy to use sterilisation apparatus is preferred by most users and many users rely upon electric steam sterilisers due to their convenience.

Electric steam sterilisers are typically stand-alone devices which may be placed on a countertop. They function by boiling water to produce steam which passes over feeding equipment contained within the device. The steam sterilises the feeding equipment killing almost all bacteria which may be present on the equipment. Once sufficient steam has been produced, and/or the steam has passed over the feeding equipment for a sufficient amount of time to sterilise the equipment, the device is turned off and its contents are allowed to cool and dry. Typical electric steam sterilisers utilise a relatively low power heating element to heat the water to produce the steam. As a result of the use of low power elements, the sterilisation process can take a considerable amount of time. Furthermore, once the sterilisation process has completed, the drying and cooling of the equipment can take a long time. Due to the nature of babies and their feeding routines, a user typically will not have a long period of time in which they are free to wait for the equipment to dry and so this can be problematic.

More sophisticated electric steam sterilising devices further include a fan which directs air into the device to help speed up the drying process. These devices offer a drying time which is reduced, when compared to the more basic device discussed above. However, despite the presence of a fan, the drying time can still be considerable, for example around 20 minutes. Often, feeding equipment is sterilised immediately prior to use and so increased drying times may be a hindrance to the feeding of a baby.

WO 2020/114397 A1 discloses a fully automatic integrated baby bottle cleaning, disinfecting, and drying machine.

EP 3 409 298 A1 discloses a device for sanitising and drying with a chamber.

CN 109 203 156 A discloses a safety negative pressure drying device for building wood.

KR 101 521 509 B1 discloses an infant feeding bottle steriliser.

The present invention, which aims to address or at least mitigate the above mentioned problems, provides an apparatus, for sterilising objects, as defined by claim 1.

The Applicant has recognised that a helical air flow formed by the at least one outlet may create an airflow within the chamber which is particularly well suited to quickly drying objects placed in the chamber. The nozzle may be shaped so as to receive an object which is to be sterilised. For example, the nozzle may be shaped and dimensioned so that a baby bottle, with the teat removed, can be placed onto the nozzle. As such, the helical air flow will pass around the object, e.g. the interior of the baby bottle, which may reduce the drying time. The Applicant has found that the apparatus according to the invention may reduce drying time significantly, for example by around 50 %, e.g. from 20 minutes down to 10 minutes in a sterilisation apparatus used for sterilising and drying baby feeding equipment. A shortened drying time may make the apparatus more suitable for its use, e.g. for sterilising and drying baby feeding equipment wherein quick turnaround times are often desired. In a set of embodiments, the apparatus is a baby equipment, e.g. baby feeding equipment, sterilising apparatus. For example, the apparatus may be suitable for sterilising objects including: baby bottles (including all of their respective parts), soothers, teethers and any other baby equipment which requires sterilisation.

A helical flow of air formed by the at least one outlet may be capable of sufficiently drying an object placed on the at least one nozzle. However, in a set of embodiments, the at least one nozzle comprises a further outlet aligned with an axis of the nozzle and arranged to direct air along the axis of the nozzle. The air directed along the axis of the nozzle thus forms an axial flow of air. The combination of an axial flow of air and a helical flow of air may be particularly well suited to drying certain objects which may be placed on the at least one nozzle. For example, in the exemplary case of an upturned bottle, the helical flow of air may act to dry the rounded side walls of the bottle, and the axial flow of air may be directed towards a base of the upturned bottle which faces the further outlet. This may therefore ensure that all of the inside of the bottle is dried.

It may be possible to create the helical flow of air by suitably shaping the outlet at the point at which the air leaves the further outlet. For example, the outlet may have a curved, e.g. helical, shape which entrains the air that passes therethrough into a helical flow. The shaping of the outlet alone may be sufficient to create a helical flow of air which maintains its helical shape as it passes out of the further outlet. In accordance with the invention. the nozzle may comprise an internal helical air flow path leading towards the outlet. The internal helical air flow path may, at least partially, entrain a portion of the air passing through the nozzle into a helical flow path as it passes towards the outlet. Entraining the air in this manner before it leaves the outlet may help to ensure that the flow of air maintains a helical shape once it has left the outlet. This may help to ensure that the helical air flow can provide a sufficient drying function once it has left the nozzle.

The helical flow path may be integrally formed within the nozzle, for example as part of a moulding of the nozzle. However, in a set of embodiments, the nozzle comprises a separate insert which defines the helical air flow path when inserted into the nozzle. As will be appreciated by those skilled in the art, integrally forming a helical flow path within the nozzle may be difficult and thus an insert which defines the helical air flow path may simplify manufacture of the apparatus. The insert may also comprise a conduit extending therethrough which provides a flow path to the further outlet. Accordingly, the insert may function to divide the air between the at least one outlet and the further outlet. Additionally, the conduit may have a larger cross-sectional area than the cross-sectional area of the further outlet. This may cause an increase in the velocity of the air passing out of the further outlet, thereby creating a strong axial flow of air. For example, the further outlet may be circular and have a smaller diameter than a diameter of the conduit which may also be circular.

The insert may be configured to only extend partly into the nozzle and define a cavity between the end of the insert and the further outlet. The nozzle may have a conical tip, and thus the cavity may also have conical shape. A conically shaped cavity may function to direct air which leaves the conduit, of the insert, towards the further outlet.

The insert may also comprise at least one location feature configured to engage with a corresponding location feature on the nozzle. This may help to ensure that the insert is positioned correctly within the nozzle, thus ensuring that the insert is capable of directing air to the at least one outlet and/or the further outlet.

In accordance with the present invention, air from the forced air flow device may be directed into the nozzle so as to flow around an internal wall of the nozzle and thereby create a helical flow of air within the nozzle. The air may be directed into the nozzle by an air ducting means, e.g. a manifold, arranged within the device. The air may be directed into the nozzle in any suitable manner such that the air flows around the internal wall of the nozzle. The internal wall of the nozzle may be suitably shaped so as to form a helical flow of air. The nozzle may be at least partially cylindrically shaped and thus the internal wall may also be at least partially cylindrically shaped. This shape may entrain the air into a helical flow. In a further set of embodiments, the air may enter the nozzle at least partially tangentially, e.g. tangentially, with the internal wall of the nozzle. The embodiments discussed above may be combined with the helical air flow path described above. Such a combination of features may advantageously form a strong helical air flow which maintains its helical shape after leaving the nozzle for an extended distance and/or extended period of time. Advantageously, however, the Applicant has recognised that introducing the air flow so as to flow around the internal wall of the nozzle to create a helical air flow may remove the need to provide a helical air flow path within the nozzle. This may simplify the manufacture of the apparatus, specifically the nozzle(s) thereof.

A single outlet may be capable of creating a helical air flow which sufficiently contributes towards reducing the drying time of the objects within the chamber. However, in a set of embodiments, the at least one outlet comprises a plurality of outlets each arranged to direct air along and around the axis so as to create a helical flow of air. Each of the plurality of outlets may comprise any of the features of the outlet described above or below. The plurality of outlets may contribute towards the formation of a strong helical air flow, which maintains its helical shape for a sufficient distance after leaving the nozzle. This may ensure that the helical air flow is capable of contributing towards quick drying of the object arranged over the nozzle. The plurality of outlets may comprise, for example, two outlets. In the exemplary case of two outlets, each of the outlets may be diametrically opposed on the nozzle. Further, in such an example, one third of the air flow through the nozzle may pass through the further outlet, one third of the air flow through the nozzle may pass through a first one of the outlets, and one third of the air flow may pass through a second of the outlets. A first of the plurality of outlets may be arranged on one side of the nozzle, and a second of the outlets may be arranged on an opposing side of the nozzle. In embodiments comprising a plurality of outlets, the outlets may be arranged in an equiangular arrangement around the nozzle.

As described above, the nozzle may be shaped and dimensioned to receive an object to be sterilised and subsequently dried. Accordingly, the number of nozzles may be dependent on the intended use of the apparatus. In a set of embodiments, the at least one nozzle comprises a plurality of nozzles. Accordingly the apparatus may be able to quickly dry a plurality of objects. For example, the apparatus may be capable of sterilising and drying a plurality of baby feeding bottles, whereby each bottle is placed over one of the plurality of nozzles. For example, in the case of an apparatus designed to sterilise six baby bottles, the plurality of nozzles may comprise six nozzles. The plurality of nozzles may ensure that the axial and helical air flow can be directed into each of the separate objects to be dried, thus providing a fast drying of each of the objects.

Whilst the nozzles may receive some objects to be sterilised and dried, other objects may be positioned elsewhere in the chamber. For example, bottles may be placed on the nozzles and other components such as teats and covers may be placed elsewhere in the chamber. As will be appreciated by those skilled in the art, once the air has passed into and around the object which is placed on the nozzle, it may then escape the object, e.g. through the opening of the object, and pass into the chamber. However, the air which passes into the chamber after passing around the object may not be capable of sufficiently drying other objects within the chamber, for example because it may already have a high moisture content following drying performed within the object. Additionally, the air speed of the air leaving the bottle may be reduced which may reduce its ability to dry other objects in the chamber. Thus, in a set of embodiments, the apparatus further comprises an air directing means, located downstream of the forced air flow device, arranged to direct a first portion of air through the at least one nozzle and a second portion of air directly into the chamber. Accordingly, the air flow from the forced air flow device is divided between the at least one nozzle and the rest of the chamber. Directing a second portion of air directly into the chamber provides a separate flow of air into the chamber, separate from the air flow from the nozzles, which is capable of drying other objects within the chamber, which are not placed on the nozzles. For example, the second portion of air may pass over other objects such as e.g. teats and covers, which are not placed on the nozzles. The air directing means may comprise any suitable arrangement that is capable of dividing the air in an appropriate manner. For example, the air directing means may comprise a plurality of channels arranged to direct the portions of air appropriately.

The relative portions of air which are directed through the at least one nozzle and which are directed directly into the chamber may depend on the application of the apparatus and the type of objects being dried therein. In a set of embodiments, however, the first portion of air comprises between 40% and 60%, preferably 50%, of the air directed by the forced air flow device. The Applicant has found that such a division of the air between the at least one nozzle, and air which is fed directly into the chamber, may be particularly well suited to the drying of objects, e.g. bottles, arranged on the at least one nozzle. This may, therefore, help to ensure that the air flow through the at least one nozzle can suitably dry the objects placed thereon.

The at least one nozzle may be a completely separate component to other parts of the apparatus, e.g. the air directing means, and be suitably connected thereto. However, in a set of embodiments, the at least one nozzle is integrally formed with the air directing means. For example, the at least one nozzle and air directing means may be formed from a single piece of moulded plastic. Integrally moulding the at least one nozzle with the air directing means in this manner may reduce the number of separate parts in the apparatus and thus speed up its manufacture and assembly. The air directing means may be in the form of a manifold.

As mentioned above, objects may be placed on the at least one nozzle. In a set of embodiments, the nozzle comprises a conical tip. The conical tip may facilitate placing of an object on the nozzle. The first outlet may be arranged at a peak of the conical tip.

As discussed above, the air flow from the at least one nozzle may act to dry objects within the chamber, specifically objects placed on the at least one nozzle. Drying of the objects may be necessary due to residual moisture left on the objects after sterilisation performed by the sterilisation means. For example, the sterilisation means may comprise an arrangement for soaking the objects in a sterilising solution. The sterilising solution may be drained from the chamber, and then the forced air flow device may be used to dry the objects within the chamber. However, this is just one form of sterilisation means and in a set of embodiments, the sterilisation means comprises a steam generator arranged to direct steam into the chamber. A steam generator may provide a quick and convenient means for sterilising the objects within the chamber, whilst avoiding the use of chemicals. Following a sterilisation process using steam from the steam generator, water which remains on the objects within the chamber may be dried by the air flow generated by the forced air flow device, in particular by the air flow generated by the at least one nozzle. Accordingly, the apparatus may be configured to operate in a sterilisation mode in which the sterilisation means is operated and a drying mode in which the forced air flow device is operated.

As discussed above, a portion of the air directed by the forced air flow device may be directed through the at least one nozzle, and a portion of the air may also be directed into the chamber. In a set of embodiments, the apparatus comprises at least one inlet, which is separate to the at least one nozzle, for allowing air directly into the chamber. The at least one inlet may be considered to allow air directly into the chamber as the inlet may not receive objects placed thereon, whereas the at least one nozzle may first direct air into an object placed thereon, rather than directly into the chamber itself. The air may subsequently pass out of the object and pass into the chamber. Of course, if there is no object placed on the nozzle, the air may pass directly into the chamber. In a set of embodiments, the apparatus comprises at least one inlet arranged to allow a portion of the air directed by the forced air flow device into the chamber, and at least one chamber outlet arranged to allow fluid to escape the chamber, wherein the at least one inlet and the at least one chamber outlet are arranged in the chamber such that at least a portion of the air passing into the chamber through the at least one inlet travels in a first direction away from the at least one chamber outlet. Accordingly, as will be appreciated, arranging the at least one inlet in a manner in which air passing into the chamber through the at least one inlet travels in a first direction away from the at least one outlet, means that in order for the air to escape the chamber via the chamber outlet, the air must first circulate, at least partially, around the chamber. The Applicant has found that the circulation achieved by this arrangement may also reduce the drying time of objects within the chamber due to the improved circulation of the air around the chamber.

A further aspect, which is not part of the claimed invention, provides an apparatus, for sterilising objects, comprising:
a chamber for housing objects to be sterilised;
a sterilising means comprising a steam generator arranged to direct steam into the chamber;
a forced air flow device arranged to direct air into the chamber;
at least one inlet arranged to allow air directed by the forced air flow device into the chamber; and
at least one chamber outlet arranged to allow fluid to escape the chamber;
wherein the at least one air inlet and the at least one chamber outlet are arranged in the chamber such that at least a portion of the air passing into the chamber through the at least one inlet travels in a first direction away from the at least one chamber outlet.

Such an apparatus has the advantage discussed above that the drying time when the forced air flow device is operated may be reduced when compared to prior art sterilising devices.

The apparatus may further comprise at least one nozzle comprising at least one outlet arranged to direct air along and around the axis of the nozzle to thereby create a helical flow of air. Further, the at least one nozzle may comprise a further outlet aligned with an axis of the nozzle and arranged to direct air along the axis of the nozzle. The at least one nozzle may have the same advantages as the at least one nozzle described above with respect to embodiments of the claimed the invention and similarly may comprise any of the features of the at least one nozzle described above with respect to embodiments of the claimed present invention.

Features will now be described which are applicable to relevant embodiments of the present invention discussed above or the apparatus disclosed for reference purposes discussed above. The at least one inlet and at least one chamber outlet may be arranged in any suitable position such that air passing through the inlet first travels in a direction away from the outlet. In a set of embodiments, the at least one chamber outlet is arranged on a lower portion of the chamber. For example, the at least one chamber outlet may be arranged on a lower portion of a sidewall of the chamber. Arranging the outlet on a lower portion of the chamber may advantageously avoid steam, and potentially heated air, escaping towards the top of the chamber. This may avoid heating of parts of the apparatus, e.g. a cover thereof, which a user is likely to come into contact with. This may, therefore, minimise the chance of harm to a user.

The at least one inlet may have any suitable form that is capable of directing air away from the at least one chamber outlet. The form of the at least one inlet may at least partially depend on the relative arrangement of the at least one inlet and the at least one outlet. For example, the at least one inlet may be angled in a direction away from the at least one chamber outlet such that air is directed away from the at least one chamber outlet. However, in a set of embodiments, the at least one inlet comprises a conduit which extends into the chamber. In embodiments comprising at least one chamber outlet arranged on a lower portion of the chamber, the conduit may conveniently extend the at least one inlet past the at least one chamber outlet, such that the air from the at least one inlet cannot bypass the rest of the chamber and pass directly out of the at least one chamber outlet. The conduit may extend to a level above the at least one chamber outlet such that air travels away from the at least one chamber outlet and is forced to circulate around the chamber before it can escape via the at least one chamber outlet. In a further set of embodiments, the conduit extends from a base of the chamber towards a central portion of the chamber. Such a set of embodiments may provide an air flow directly to objects stored higher up in the chamber, and thereby assist in the quick drying of such objects.

Irrespective of the form of the at least one inlet and the at least one chamber outlet, the at least one inlet and at least one chamber outlet may have any suitable relative positioning in the chamber such that the air from the at least one inlet is directed away from the at least one chamber outlet. In a set of embodiments, the at least one inlet is arranged above the at least one chamber outlet. Arranging the at least one inlet above the at least one chamber outlet may advantageously further minimise the amount of air which bypasses the chamber and passes directly out through the at least one chamber outlet. As will be appreciated by those skilled in the art, air flows travelling towards the at least one chamber outlet may pull surrounding air flows towards the at least one chamber outlet. Thus, arranging the at least one inlet above the at least one chamber outlet may minimise the amount of air which can be pulled towards the at least one chamber outlet by other air flows within the apparatus. This may therefore ensure that the air from the at least one inlet properly circulates around the chamber, and thus dries any objects placed therein, before passing out through the at least one chamber outlet. This may be particularly important when the flow rate of air from the forced air flow device is smaller, e.g. due to a low speed fan which may be desired to minimise the noise of the apparatus.

The air which passes into the chamber in a direction away from the at least one chamber outlet may advantageously be caused to circulate within the chamber before it can escape through the chamber outlet. In a set of embodiments, a portion of the chamber facing the at least one air inlet is shaped so as to redirect air around the chamber towards the at least one air outlet. In such embodiments, the air flow may be more efficiently directed around the chamber which may further improve the circulation of air, which may improve the drying action of the air. For example, an upper wall of the chamber may be curved to direct the air flow towards a lower portion of the chamber. By shaping a portion of the chamber to direct the air flow, the circulation of the air may be controlled in a desired manner, which may involve directing the air over certain regions in the chamber which may otherwise have not received a strong air flow.

In a set of embodiments, the at least one chamber outlet is configured to create an increased pressure within the chamber. This may be achieved by any suitable means. In a set of embodiments, a cross-sectional area of the at least one chamber outlet is less than a cross-sectional area of an air supply inlet to the forced air flow device. As will be appreciated by those skilled in the art, having the cross-sectional area of the at least one chamber outlet being less than the cross-sectional area of the air supply inlet, may cause the inside of the chamber to become pressurised during use as the at least one air outlet may restrict the flow of air out of the chamber. Restricting the flow of air out of the chamber in this manner may cause increased circulation of the air around the chamber before it is able to escape. This increased circulation may reduce the drying time for objects in the chamber. Additionally, air which is under increased pressure may be capable of holding more moisture, and therefore may more efficiently dry objects within the chamber. In embodiments wherein there are a plurality of chamber outlets, the cross-sectional area of the plurality of chamber outlets may be the sum of the cross-sectional area of each of the chamber outlets.

The forced air flow device may be capable of forcing a large volume of air into the chamber and thus the flow rate of air out of the chamber, specifically the at least one chamber outlet, may be relatively large. As a result, a steady flow of air and/or steam may be forced out of the chamber through the at least one chamber outlet. The flow of air and/or steam out of the at least one chamber outlet may be even stronger in embodiments wherein the at least one chamber outlet has a cross-sectional area which is smaller than the cross-sectional area of the air supply inlet through which air is drawn by the forced air flow device. A strong flow of air and/or steam out of the chamber may cause harm to a user or the environment in which the apparatus is placed. Thus, in a set of embodiments, the apparatus further comprises an expansion chamber, arranged downstream of the at least one chamber outlet, which comprises at least one apparatus outlet in fluid communication with the environment in which the apparatus is arranged. The expansion chamber may disrupt the flow of air and/or steam out of the at least one chamber outlet which, as discussed above, may be pressurised due to the relative size of the at least one chamber outlet. The at least one apparatus outlet may be arranged in a suitable position such that air and/or steam are directed out of the apparatus in a region in which a user is less likely to place other objects. This may, advantageously, help to prevent any harm being caused to a user of the apparatus or to objects in the vicinity of the apparatus.

In a further set of embodiments, the at least one apparatus outlet has a cross-sectional area which is larger than a cross-sectional area of the at least one chamber outlet. The increased cross-sectional area of the at least one apparatus outlet, at least when compared to the cross section of the at least one chamber outlet, may thus allow air to escape the expansion chamber, and into the environment into which the apparatus is arranged, in a more diffuse manner. This may therefore avoid a strong flow of air and/or steam from being output from the apparatus which may otherwise potentially cause harm to users or objects in the environment which the apparatus is placed. The at least one apparatus outlet may comprise a plurality of apparatus outlets. In embodiments comprising a plurality of apparatus outlets, the cross-sectional area of the plurality of apparatus outlets may be the sum of the cross-sectional areas of each of the apparatus outlets. Similarly, in embodiments comprising a plurality of chamber outlets, the cross-sectional area of the plurality of chamber outlets may be the sum of the cross-sectional area of each of the plurality of chamber outlets.

In embodiments comprising a steam generator, the steam generator may have any suitable configuration that is capable of producing steam. In a set of embodiments, the steam generator comprises a heating element arranged to heat a heated base arranged below the chamber. Such an arrangement may provide a convenient arrangement of the steam generator which is capable of producing steam which may permeate upwards through the chamber. The heated base may comprise a well at its centre for containing water. As such, a user may simply pour water into the chamber, which may drain into the well, in order to supply the steam generator with water for the production of steam.

In order to efficiently dry the objects within the chamber, it may be desirable to direct 'fresh' air into the chamber, i.e. air which has not already circulated around the chamber. Thus, in a set of embodiments, the forced air flow device is arranged to direct air, from the environment in which the apparatus is placed, into the chamber. Accordingly, the forced air flow device acts to direct air from outside of the apparatus, i.e. fresh air, into the chamber. The air from outside of the apparatus may be less saturated with moisture, and thus be able to dry the objects within the apparatus more quickly. Air may be drawn into the apparatus by the forced air flow device through an air supply inlet which is in fluid communication with the environment in which the apparatus is placed. The air supply inlet may be spaced from an apparatus outlet such that the forced air flow device does not immediately recirculate air which has passed out of the apparatus. For example, the air supply inlet and apparatus outlet may be arranged on different sides of the apparatus, or on an upper part and lower part. Similarly, the at least one apparatus outlet may be arranged to direct air away from the air supply inlet so that the air escaping the apparatus is not immediately drawn back into the apparatus.

The forced air flow device may comprise any suitable device for forcing air into the chamber. In a set of embodiments, the forced air flow device comprises an electric fan. The electric fan may be a D.C. fan, however, preferably the electric fan is an A.C. fan as this may remove the need to provide a D.C. power supply within the apparatus. An electric fan is not the only type of forced air flow device which may be used, and in alternative examples the forced air flow device may comprise an air pump devoid of a fan, for example a positive displacement pump.

As discussed above, by appropriately controlling the air flow within the chamber, the drying time may be significantly reduced. The drying time for objects within the chamber may also be accelerated by heating the air in the chamber. This may be achieved by any suitable means. For example, in embodiments comprising a steam generator which comprises a heated base, the heated base may continue to be heated following the production of steam, to heat air which flows into the chamber. However, in a set of embodiments, the apparatus further comprising an electric heating element arranged to heat the air directed by the forced air flow device.

The electric heating element may be arranged at any suitable position within the apparatus such that is capable of heating the air within the chamber. For example, the heating element may be arranged in the chamber and be exposed to the air within the chamber, e.g. an immersed heating element. Alternatively, the heating element may be provided directly in the air flow directed by the forced air flow device, i.e. immediately downstream of the forced air flow device. Advantageously, in this arrangement, all of the air directed by the forced air flow will be heated. The electric heating element may be of any suitable type, e.g. a sheathed heating element, or a coiled-wire heating element. By providing a heating element to heat the air within the chamber, the temperature of the air which is circulated around the chamber by the forced air flow device will be increased and thus due to the increased temperature any condensed water on the objects within the apparatus may dry off more quickly.

Particularly in embodiments comprising a steam generator in which steam permeates through the chamber, it may be desirable to prevent any moisture from being able to reach the forced air flow device which may comprise exposed electrical components. Thus, in a set of embodiments, the apparatus further comprises a one-way valve arranged downstream of the forced air flow device and configured to allow air to flow through the one-way valve towards the chamber. The one-way valve may thus allow air to flow from the forced air flow device towards the chamber, whilst at the same time preventing air, or any other fluid, from travelling towards the forced air flow device. This may ensure the safety of the apparatus as it may prevent any moisture from reaching the forced air flow device which may cause damage to any electrical components of the forced air flow device. The one-way valve may be provided by any suitable means. For example, the one-way valve may comprise a resiliently biased flap valve, wherein the resilient bias can be overcome by the air flow from the forced air flow device. The resiliently biased valve may be biased into a closed position when the forced air flow device does not operate to produce an air flow.

The apparatus may comprise any suitable means for controlling its operation. For example, the apparatus may comprise a control arrangement. The control arrangement may comprise an electronic and/or a thermomechanical arrangement configured to control operation of the apparatus. For example, the electronic and/or thermomechanical arrangement may be configured to start and stop operation of the forced air flow device. The forced air flow device may, for example, be operated for a fixed period of time. In embodiments comprising a steam generator, the electronic and/or thermomechanical arrangement may be configured to stop operation of the steam generator once a threshold temperature is detected. The threshold temperature may be indicative of all of the water having been evaporated to form steam. The electronic arrangement may comprise a thermistor arranged to sense the temperature of part of the apparatus.

As the primary purpose of the apparatus is to sterilise objects placed in the chamber, ensuring that the chamber remains sterile, particularly after sterilisation has occurred, is important. As will be appreciated, there is a possibility that directing air into the chamber in the drying mode will increase the risk of contaminating the sterilised objects inside the apparatus through the passing of airborne bacteria. Therefore, in a set of the embodiments, the apparatus further comprises an air filter arranged to filter the air directed by the forced air flow device. The air filter may be any suitable filter, for example a high efficiency particulate air (HEPA) filter.

The air filter may be arranged upstream of the forced air flow device. However, in an advantageous set of embodiments, the air filter is arranged downstream of the forced air flow device. The Applicant has recognised that arranging the air filter downstream of the forced air flow device is advantageous for multiple reasons. For example, it ensures that all of the air directed by the forced air flow device into the chamber is filtered. Further, in this downstream position, the air filter can also act as a baffle to prevent steam escaping the chamber through the forced air flow device during a sterilisation process. Preventing steam from passing back through the forced air flow device helps to ensure that the steam remains in the chamber thereby sterilising the contents, and also it helps to prevent moisture from reaching the forced air flow device and thus avoids any moisture based damage to its electrical components. Additionally, steam which passes over the air filter will also cause the air filter to heat up which may, advantageously, sterilise the air filter itself.

In embodiments comprising a steam generator, the steam generator may be capable of accommodating 50-150 ml of water for boiling in a sterilisation mode. For example, the steam generator may be capable of accommodating 100 ml of water for boiling in a sterilisation mode.

As discussed above, some objects to be sterilised may be arranged on top of the nozzles so that air from the first outlet and further outlet dries the inside of the objects placed thereon, and other objects may be arranged elsewhere in the chamber and dried by airflow around the rest of the chamber, e.g. from the at least one inlet. In a set of embodiments, the apparatus further comprises a support structure for supporting objects within the chamber. The support structure may be arranged above and be spaced from the at least one nozzle such that there is space for an object to be placed on top of the nozzle. The support structure may advantageously allow more objects to be placed in the chamber, thus facilitating the sterilisation and drying of more objects and thereby increasing the efficiency of the apparatus.

In a set of embodiments, the support structure comprises at least one shelf member arranged within the chamber to support the objects away from a base of the chamber. A shelf member may provide a convenient means for supporting the objects within the chamber. In a further set of embodiments, the support structure comprises at least one support feature to assist in the support of objects thereon. The at least one support feature may allow for the appropriate positioning of objects on the support structure, which may help to ensure that they are properly sterilised and dried.

In a further set of embodiments, the one or more of the support features may be provided with an aperture therethrough. In a similar manner to the nozzle described above, an aperture within the support features may allow fluid, e.g. air, to pass therethrough directly into or onto an object, thereby improving the sterilising or drying of the object.

The at least one support member may extend across only part of the cross section of the chamber, and thus fluid such as steam and air may pass around the support member and around any objects arranged on the support structure. However, in a set of embodiments, the at least one support member comprises at least one aperture arranged to permit the passage of fluid therethrough. Such a support structure may allow fluid, such as steam and air, to permeate around the objects on the support structure more easily, and thus improve the sterilisation and drying of the objects. Such an at least one aperture may also allow the support structure to extend across the entire cross-section of the chamber, thus providing more space to arrange objects to be sterilised.

Some preferred embodiments of the present invention will now be described, by way of example only, and with reference to the accompanying drawings, in which:
Fig. 1 shows a perspective view of an apparatus in accordance with an embodiment of the invention;
Fig. 2 shows a perspective view of the apparatus shown in Fig. 1 with a cover removed;
Fig. 3 shows a perspective view of the rear of the apparatus shown in Fig. 2;
Fig. 4 shows a perspective view of the apparatus shown in Fig. 1 with part its body removed to reveal its internal components;
Fig. 5 shows a view of the underside of the manifold which directs air into the nozzles and air inlets;
Fig. 6 shows the manifold shown in Fig. 5 with a cover thereof removed to reveal the internal structure of the manifold;
Fig. 7 shows a cut-away view through the manifold to show the internal structure of the nozzles;
Fig. 8 shows a further cutaway view through the manifold to show further internal structure of the nozzles;
Fig. 9 shows a perspective view of an insert which may be inserted into each of the nozzles;
Figs. 10A-D show a cut-away views of the apparatus shown in Fig. 1, showing the forced air flow device, the one-way valve and the heating element arranged to heat the air flow from the forced air flow device;
Fig. 11 shows a cutaway view of the apparatus shown in Fig. 1, showing an expansion chamber;
Fig. 12 shows a perspective view of a steam generator of the apparatus of Fig. 1;
Fig. 13 shows a perspective view of the underside of the steam generator shown in Fig. 12;
Fig. 14 illustrates the flow of air within the chamber of the apparatus from the air inlets to the chamber outlets;
Fig. 15 illustrates the flow of air out of the first and further outlets on the nozzles;
Fig. 16 shows a perspective view of an apparatus in accordance with another embodiment of the present invention;
Fig. 17 shows the apparatus shown in Fig. 16 with its door removed to reveal its internal components;
Fig. 18 shows another perspective view of the apparatus shown in Fig. 16 with the internal support structures removed;
Fig. 19 shows a cutaway view of the apparatus shown in Fig. 16 to reveal an expansion chamber within the apparatus;
Fig. 20 shows a perspective view of a manifold of another embodiment of the present invention;
Fig. 21 shows a plan view of the underside of the manifold shown in Fig. 20; and
Fig. 22 shows a perspective view of the underside of the manifold shown in Fig. 20.

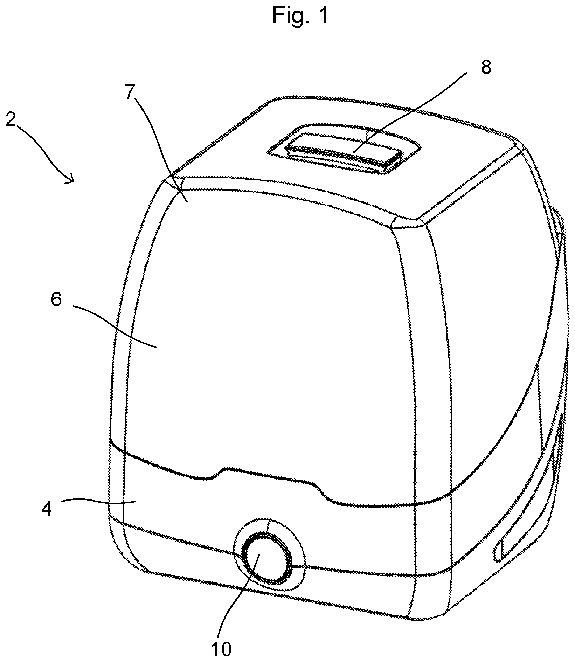

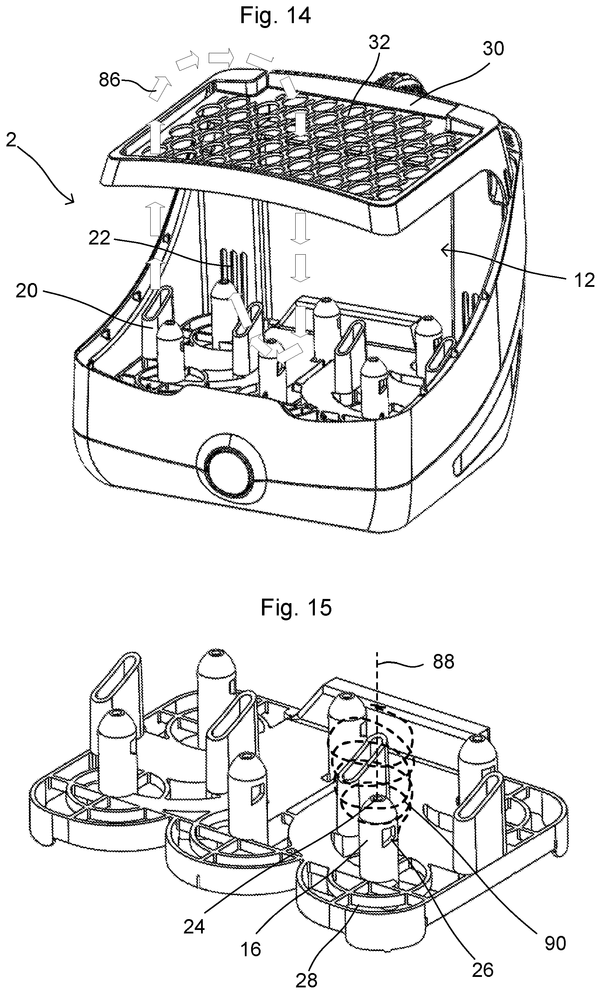

Features of an apparatus 2 will first be described with reference to Figures 1 to 13 and operation of the apparatus 2 will then be described with reference to Figures 14 and 15, as well as Figures 1 to 13. Figure 1 shows a perspective view of an apparatus 2, for sterilising objects, in accordance with an embodiment of the present invention. The apparatus 2 may be a baby equipment, e.g. baby feeding equipment, sterilising apparatus and be capable of sterilising any baby equipment which requires sterilisation. The apparatus 2 comprises a body 4 on which a cover 6 is arranged. Whilst the cover 6 is shown as opaque, the cover 6 may be made from a transparent or translucent material so that a user can see objects being sterilised within the apparatus 2. A handle 8 is provided on the top of the cover 6 for use in separating the cover 6 from the base 4. A power button 10 is arranged on the front of the base 4 and may be used to switch the apparatus 2 on and off. The cover 6 comprises a curved upper portion 7 which may help to direct air within the apparatus 2.

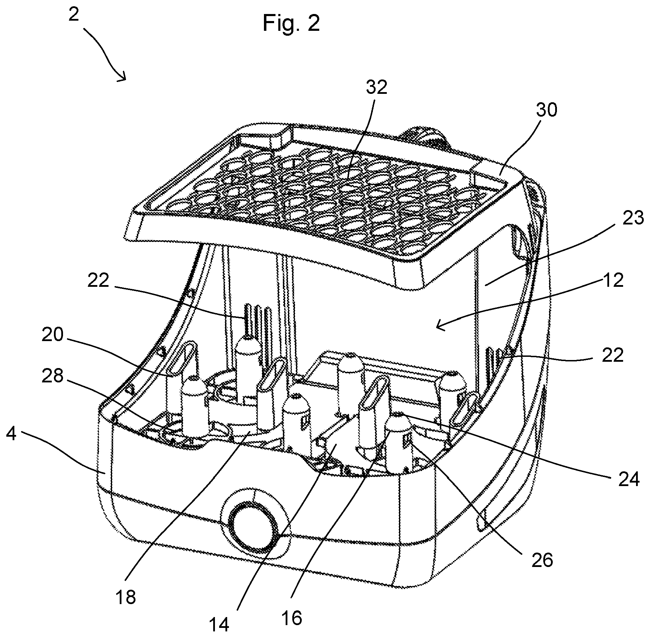

Figure 2 shows a view of the apparatus 2 shown in Figure 1, with the cover 6 removed to reveal internal components of the apparatus 2. The cover 6 (no longer visible in this Figure) together with the body 4 define a chamber 12 in which objects to be sterilised may be placed. At the base of the chamber 12 is a manifold 14 which comprises a plurality of nozzles 16 for directing air from a forced air flow device (not visible in this Figure) into objects within the chamber 12. In the exemplary embodiment depicted there are six nozzles 16. The nozzles 16 are integrally formed with the manifold 14, i.e. the manifold and nozzles 16 are made from a single piece of material. As will be described in more detail below, objects may be placed onto the nozzles 16 for sterilisation and drying within the chamber 12.

The manifold 14 further comprises openings 18 which allow steam to pass into the chamber 12 from a steam generator (not visible in this Figure) which is arranged below the manifold 14. The manifold 14 also comprises a plurality of air inlets 20 which direct air from the forced air flow device into the chamber 12. The air inlets 20 are in the form of conduits which extend upwards towards the centre of the chamber 12. In the exemplary embodiment depicted, there are four air inlets 20. Unlike the nozzles 16 which are configured to allow objects to be placed thereon, the air inlets 20 are simply configured to allow air to pass directly into the chamber 12. Similarly to the nozzles 16, the inlets 20 may be integrally formed with the manifold 14. As will be appreciated by those skilled in the art, the apparatus 2 may comprise any number of nozzles 16, and any number inlets 20, depending on the intended use of the apparatus 2.

At the rear of the chamber 12 are chamber outlets 22 which allow steam and air to escape the chamber 12 during use of the apparatus 2. As depicted, the chamber outlets 22 are arranged towards a lower portion of the chamber 12, and are on the back wall 23 of the body 4. In the embodiment depicted, there are two chamber outlets 22, however it will be appreciated that any number of chamber outlets 22 may be provided. As will be described in more detail later with reference to Figure 14, the inlets 20 and chamber outlets 22 are arranged such that at least a portion of the air passing into the chamber 12 through the inlets 20 travels in a direction away from the chamber outlets 22. In this case, the air passing into the chamber 12 will initially travel in an upward direction away from the chamber outlets 22.

Each of the nozzles 16 comprises a further outlet 24 which is aligned with an axis of the nozzle 16 and an outlet 26 which is arranged to direct air along and around the axis of the nozzle 16 to thereby create a helical flow of air. Around the base of each nozzle 24 are a plurality of openings 28 in the manifold 14 that allow steam to pass into an object in a sterilisation mode when it is placed on one of the nozzles 16, and also allow air and steam to pass out of the object in a drying mode when air is forced into the object through the nozzle 16.

A support structure 30 is arranged in an upper portion of the chamber 12. The support structure 30 may support objects within the chamber 12 during a sterilisation process. The support structure 30 comprises a plurality of openings 32 which allow the passage of steam and air through the support structure 30 so as to reach the objects supported thereon. In the exemplary case of sterilising feeding equipment, the support structure 30 may support bottle teats and covers.

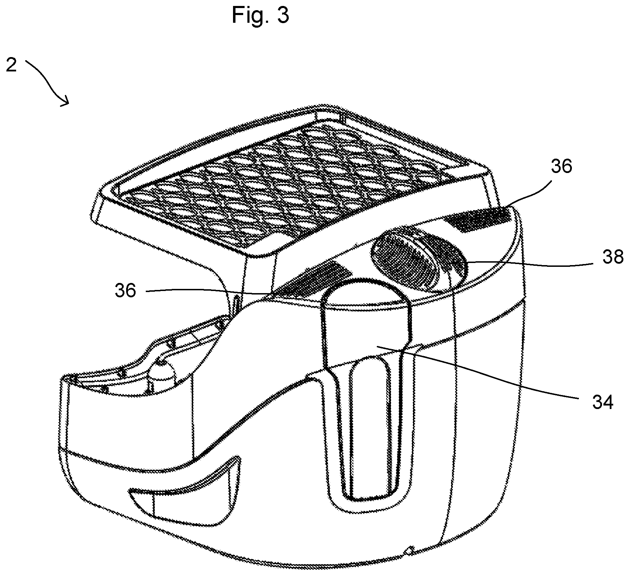

Figure 3 shows a perspective view of the rear of the apparatus 2. As shown, the apparatus 2 comprises a measuring jug 34 which may be used by a user to measure out the volume of water required by the apparatus 2 to perform a sterilisation process. The measuring jug 34 may thus be detachable from the apparatus 2. The measuring jug may, for example, be configured to measure between 50-150 ml, e.g. 50 ml of water. The apparatus 2 also comprises apparatus outlets 36 which allow air and steam to escape the apparatus 2 into the surrounding environment in which the apparatus 2 is placed. Additionally, the apparatus comprises an air supply inlet 38 into which air is drawn, from the environment in which the apparatus 2 is placed, by the forced air flow device (not visible in this Figure).

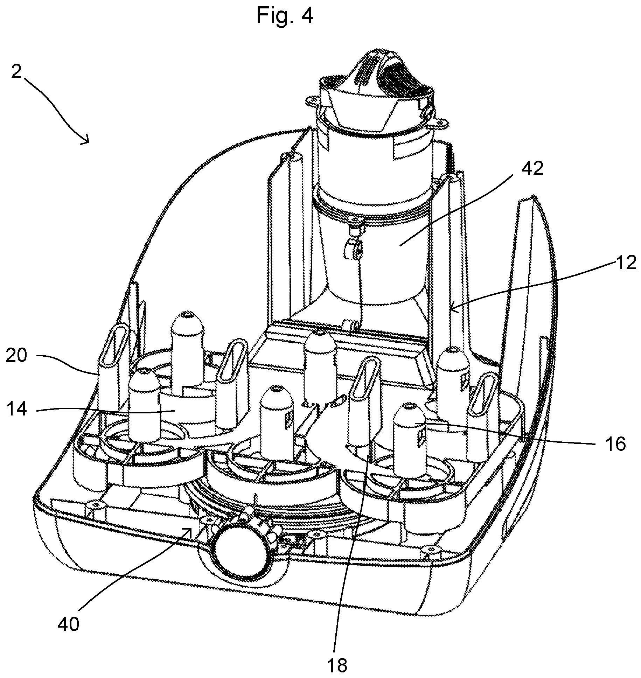

Figure 4 shows a perspective view of the apparatus 2 with part of its body 4 removed to reveal further internal components of the apparatus 2. As visible in Figure 4, a steam generator 40 is arranged beneath the manifold 14. As such, in use, steam from the steam generator 40 will pass up through the openings 18 in the manifold 14, into the chamber 12 and around the objects within the chamber 12 thereby sterilising the objects. A forced air flow device (not visible in this Figure) is contained within ducting 42 which is arranged to direct air from the forced air flow device into the manifold 14 and out through the nozzles 16 and air inlets 20.

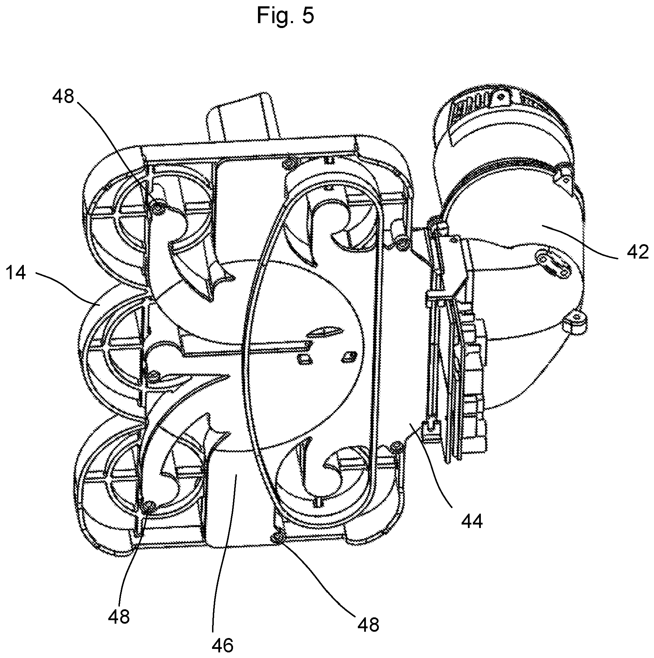

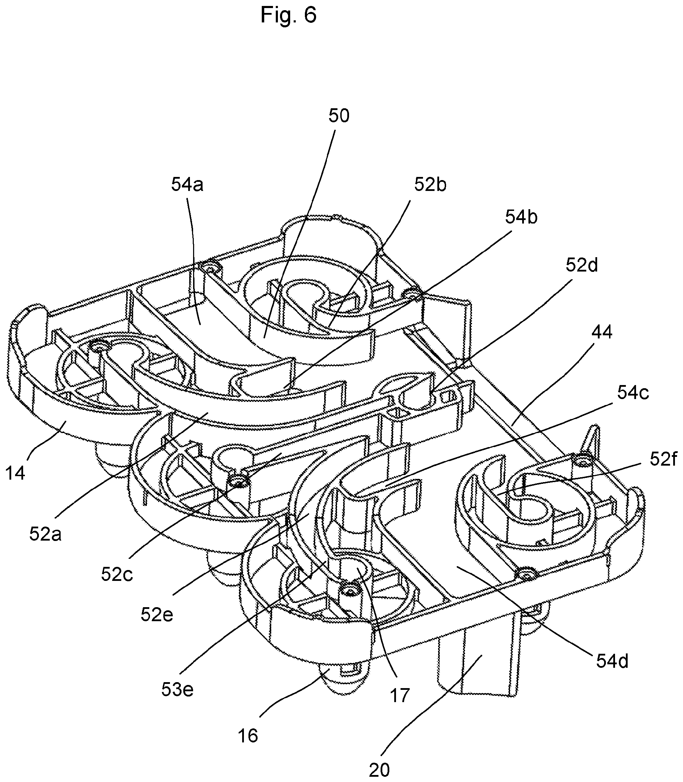

Figure 5 shows a perspective view of the underside of the manifold 14. The ducting 42, which directs air from the forced air flow device, feeds into an inlet portion 44 of the manifold 14. A cover 46 is attached to the manifold 14 via plurality of screws 48. The cover 46 acts to enclose an internal structure of the manifold 14 such that all of the air from the forced air flow device passes out through the nozzles 16 and air inlets 20. Figure 6 shows the manifold 14 with the ducting 42 and cover 46 removed to reveal the internal structure of the manifold 14. As shown in this Figure, the manifold 14 comprises internal walls 50 which define channels 52a-52f that direct air towards the each of one of the six nozzles 16. The internal walls 50 also define further channels 54a-54d which direct air to each of the four air inlets 20.

Air which passes through the inlet portion 44 is divided by the internal walls 50 into each of the channels 52a-52f and further channels 54a-54d. The internal walls 50 of the manifold 14 therefore act as an air directing means which is located downstream of the forced air flow device. A first portion of the air from the forced air flow device is directed through the nozzles 16, via the channels 52a-52f, and a second portion of the air is directed through the air inlets 20, via channels 54a-54d. The positioning of the internal walls 50 and thus the size of the channels 52a-52f and further channels 54a-54d defines the respective ratio of the first and second portions.

With reference to the nozzle 16 in the bottom left of Figure 6, as shown, the channel 52e comprises a channel outlet 53e which directs air so as to flow around an internal wall 17 of the nozzle 16. Directing the air in the manner depicted may help to form a helical flow of air within the nozzle 16. In the embodiment depicted, the nozzle 16 has a cylindrical shape and thus the internal wall 17 of the nozzle 16 also has a cylindrical shape. The air may be considered to be introduced, by the channel outlet 53e, at least partially tangentially to the internal wall 17. Once the air is inside the nozzle 16, and the helical air flow is at least partially formed, the helical flow of air may continue to be entrained by the helical air flow path as will be described below. The other channel outlets of the other channels 52a, 52b, 52c, 52d, 52f may direct air into their respective nozzles in a similar manner.

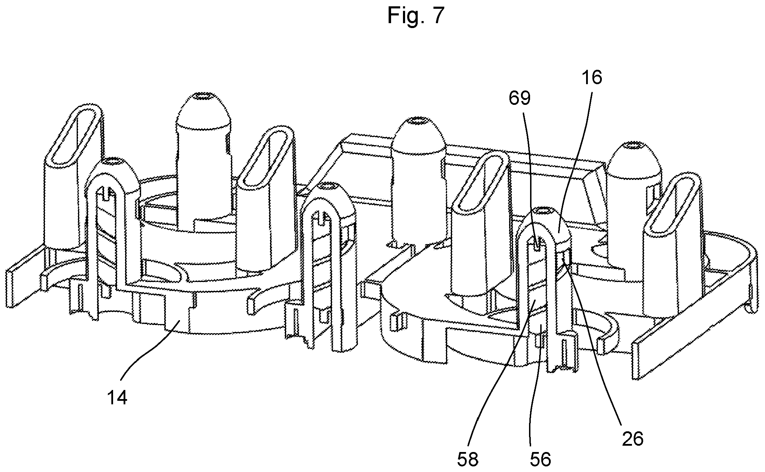

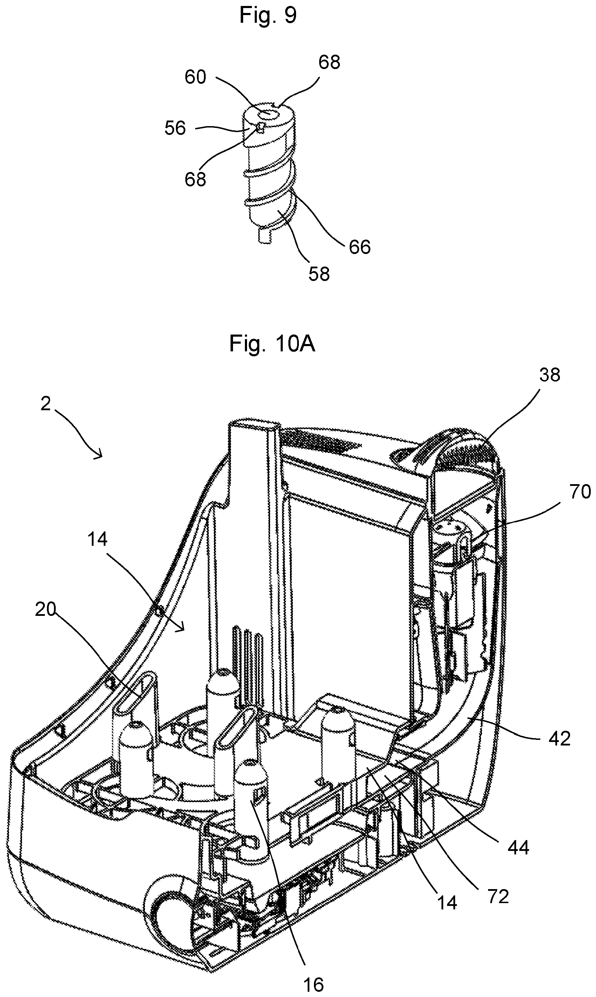

Figure 7 shows a cutaway view through the manifold 14 to reveal the internal structure of the nozzles 16. As visible in this cutaway view, the nozzle 16 comprise an insert 56 arranged therein which defines a helical flow path 58 which leads up to the outlet 26. The helical flow path 58 may act to entrain the air into a helical flow as it passes through the nozzle 16 such that as it leaves the outlet 26 the air maintains a helical flow for a substantial distance. The nozzle comprises protrusions 69 which engage with corresponding recesses 68 (shown in Figure 9), for use in properly locating the insert 56 in the nozzle 16. Of course, the insert 56 which defines the helical flow path 58 may not be necessary, and the outlet 26 alone, and/or in combination with channel outlet 53e which introduces the air in a manner such that it flows around the internal wall 17 of the nozzle, may be capable of imparting a helical flow to the air as it leaves the outlet 26 of the nozzle 16. Whilst the helical flow path 58 is defined by a separate insert 56 which is inserted into each of the nozzles 16, the helical flow path 58 may be integrally formed with the nozzle 16 itself, thus removing the need for a separate insert 56.

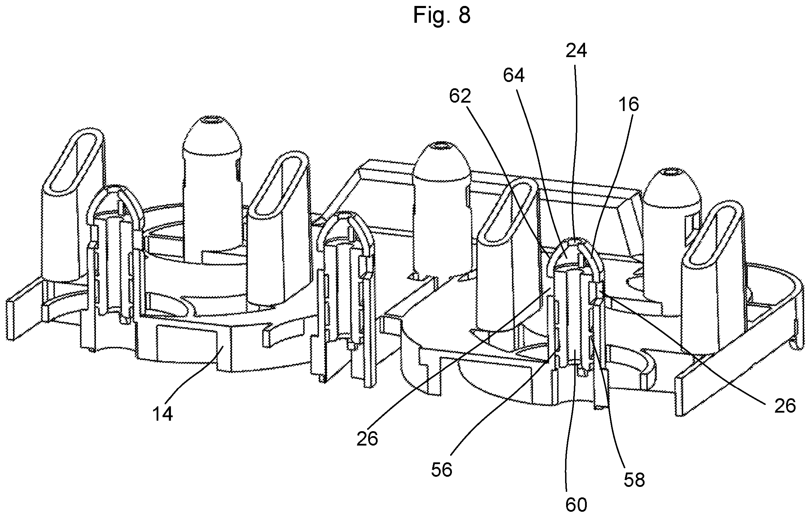

Figure 8 shows a further cutaway view through the manifold 14. As shown, the insert 56 comprises a conduit 60 extending therethrough that directs air towards the further outlet 24. The insert 56 is thus capable of directing air to the further outlet 24 and the outlets 26. The nozzle 16 has a conical tip 62 which defines a corresponding internal conical cavity 64 at the conical tip 62 of the nozzle 16. The insert 56 only extends part way into the nozzle 16 and does not fill the internal conical cavity 64. The cross-sectional area of the conduit 60 is greater than the cross-sectional area 24 of the nozzle 16. As a result, air is able to pass up through the conduit 60, into the internal conical cavity 64 whereby it is focussed towards the further outlet 24. This acts to increase the speed of the air as it passes out of the further outlet 24 thereby creating a strong flow of air which travels along the axis of the nozzle 16. As is also visible in Figure 8, the nozzle 16 may comprise a plurality, in this case two, outlets 26. In the exemplary case depicted, the outlets 26 are arranged in a diametrically opposed manner on the nozzle 16. The helical flow path 58 may direct air towards each of the outlets 26. The multiple outlets 26 may contribute towards the formation of a strong helical flow of air.

Figure 9 shows the insert 56 in isolation. As visible, the insert 56 comprises the conduit 60 and helical flow path 58. The helical flow path 58 is defined by a helical wall 66 which extends around the length of the insert 56. The insert 56 comprises two recesses 68 on an upper portion thereof. The two recesses 68 may engage with corresponding protrusions 69 (visible in Figure 7) and thus function to ensure the proper location of the insert 56 within the nozzle 16. Whilst two recesses 68 are shown, any number may be included. Similarly, any other location means may be provided for assisting with the location of the insert 56 within the nozzle 16.

Figure 10A shows a cutaway view through the apparatus 2 to reveal the forced air flow device 70 arranged within the ducting 42. The forced air flow device 70 in the embodiment depicted is in the form of an electric fan. The forced air flow device 70 draws air into the apparatus 2, from the environment in which the apparatus 2 is located, through the air supply inlet 38. The forced air flow device 70 forces air through the ducting 42 into an internal volume 72 of the manifold 14 and out through the nozzles 16 and air inlets 20 into the chamber 12. There may be a filter arranged upstream or downstream for filtering the flow of air into the apparatus 2.

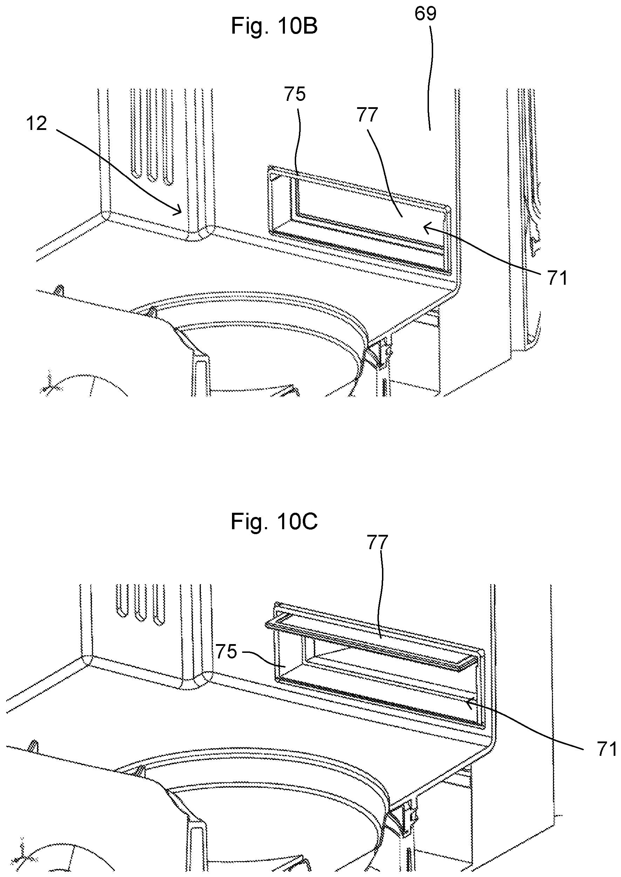

Figure 10B shows a cut-away view of the apparatus 2 with the manifold 14 removed. As shown in this Figure, a one-way valve 71 is arranged in a back wall 69 of the chamber 12. The one way valve 71 is arranged at the end of the ducting 42 (not visible in this Figure), immediately before the inlet portion 44 of the manifold 14 (not visible in this Figure). Of course, the one-way valve 71 could be arranged in any other suitable position. The one way valve 71 comprises a valve member 77, in the form of a pivotally mounted flap, arranged in a valve opening 75. In the embodiment depicted, the valve opening 75 has a rectangular shape to match the rectangular shape of the inlet portion 44 of the manifold 14.

In the position shown in Figure 10B, the valve member 77 is in a closed position in which the valve opening 75 is closed and thus fluid cannot flow through the one-way valve 71. The valve member 77 may be resiliently biased into this closed position. For example, a spring may bias the valve member 77 into the closed position. In addition, or alternatively, the valve member 77 may tend towards the closed position under the action of gravity. When in this closed position, steam and air may be prevented from passing through the one-way valve 71 into the ducting 42 and towards the forced air flow device 70.

Figure 10C shows the one-way valve 71 with the valve member 77 in an open position. In this position, air can freely flow through the valve opening 75 from the forced air flow device 70. The valve member 77 may be moved from the closed position shown in Figure 10B into the open position shown in Figure 10C under the force of the air directed by the forced air flow device 70. The force of the air may be capable of overcoming any bias that tends to move the valve member 77 towards the closed position. Accordingly, the one-way valve 71 may automatically open upon operation of the forced air flow device 70.

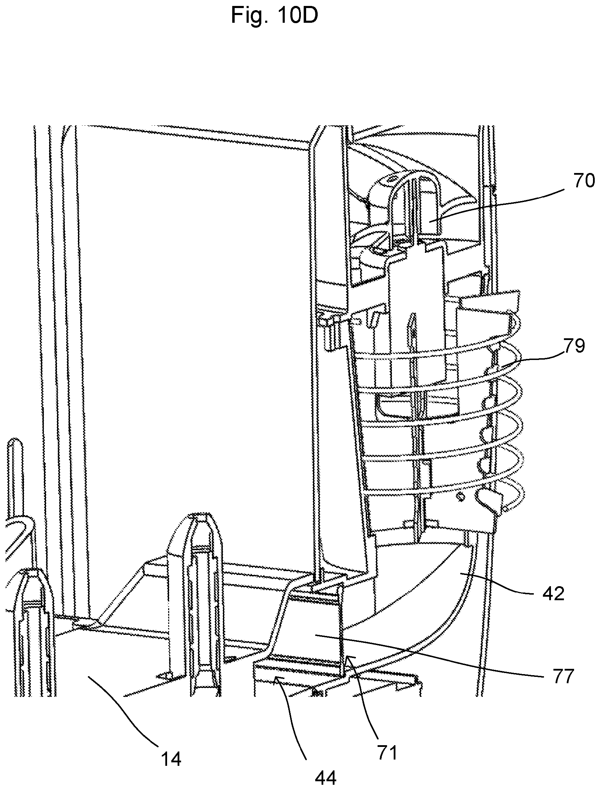

Figure 10D shows another cut-away view of the apparatus 2, focussing on the ducting 42. As shown in this Figure, the apparatus 2 further comprises a heating element 79 arranged in the flow path downstream of the forced air flow device 70. The heating element 79 is in the form of a coiled resistance wire configured to generate heat upon the flow of electricity therethrough. Of course, any other suitable heating element may be provided. The heating element 79 acts to heat air directed from the forced air flow device 70 such that the air which is forced into the chamber 12 is heated. Heating the flow of air into the chamber 12, may further reduce the drying time for objects within the chamber 12. This Figure also more clearly shows how the one way valve 71 is arranged in the flow path between the ducting 42, and hence the forced air flow device 70, and the inlet portion 44 of manifold 14.

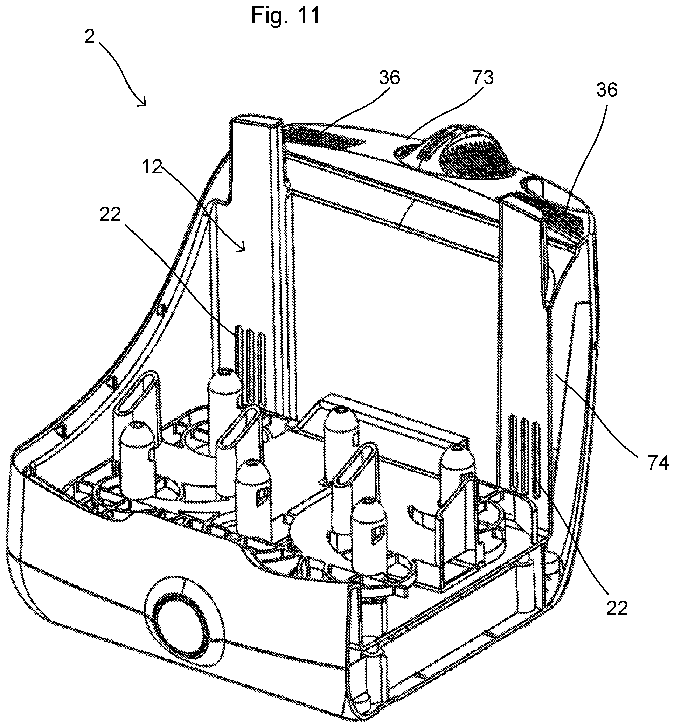

Figure 11 shows another cut-away view through the apparatus 2. As is visible in this Figure, the apparatus 2 comprises an expansion chamber 74 arranged in a rear portion 73 of the apparatus 2 where the forced air flow device 70 and ducting 42 are arranged (not visible in this Figure). The chamber outlet 22 in the chamber 12 is in fluid communication with the expansion chamber 74. As a result, during operation, steam and air may pass out of the chamber 12 into the expansion chamber 74. Steam and air within the expansion chamber 74 may subsequently pass out of the expansion chamber 74 via the apparatus outlet 36. The apparatus outlet 36 may have a larger cross-sectional area than the chamber outlet 22 such that the steam and air may pass out of the expansion chamber in a more diffuse manner than the steam and air passing out of the chamber 12 via the chamber outlets 22. This may help to avoid a jet of steam and/or air which may cause damage or harm to an individual or objects within the vicinity of the apparatus 2. Whilst only the expansion chamber 74 on the right-hand side of the apparatus 2 is visible in this Figure, it will be appreciated that a similar expansion chamber 74 is present on the other side of the apparatus and is in communication with the other apparatus outlet 36 on the left-hand side of the chamber 12. The expansion chambers 74 may be independent chambers not in fluid communication with one another, or alternatively they may be formed as a single chamber.

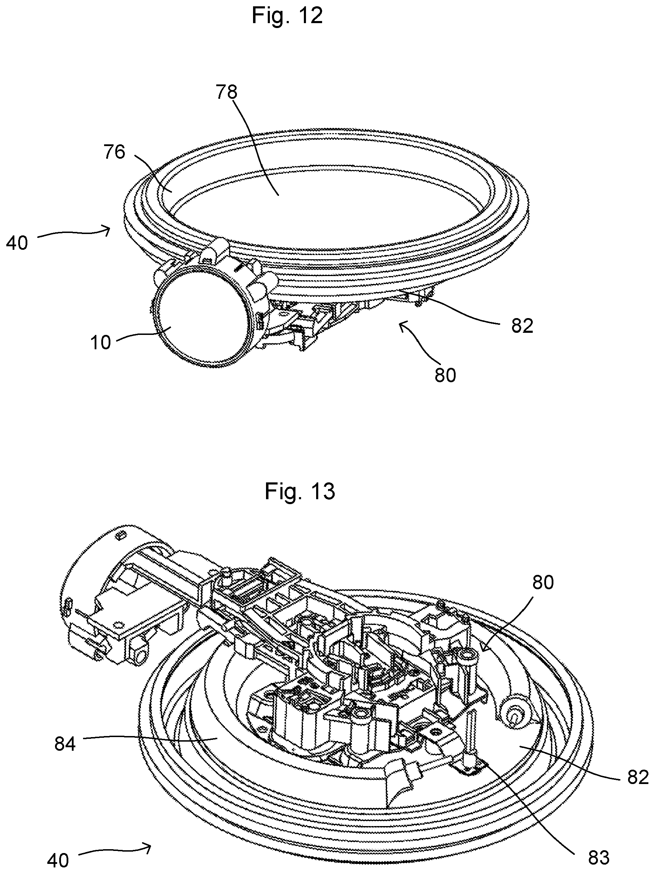

Figure 12 shows the steam generator 40 in isolation. The steam generator 40 comprises a heated plate 76 which is formed to define a well 78 at its centre. The well 78 serves to contain water, in use, which is heated to produce steam for sterilising objects within the apparatus 2. A control arrangement 80 is coupled to a base 82 of the heated plate 76, and is operatively coupled to the switch 10. Figure 13 shows a view of the underside of the steam generator 40. As shown in Figure 13, the control arrangement 80 is arranged in contact with the base 82 of the steam generator 40. The control arrangement 80 may be in the form of the Applicant's U12-Series control. A sheathed heating element 84 is also in contact with the base 82 and functions to heat the base 82, and thus any water contained within the well 78 shown in Figure 12. The control arrangement 80 may selectively supply electrical power to the sheathed heating element 84. The control arrangement 80 depicted is a thermomechanical arrangement for controlling the supply of electrical power to the sheathed heating element 84. The control arrangement 80 may thus comprise a thermally sensitive element, e.g. a thermally sensitive actuator, arranged to monitor the temperature of the heated plate 76. Of course, alternatively, the control arrangement 80 may comprise electronic means for monitoring the temperature of the heated base 82 and for controlling the supply of power to the sheathed heating element 84.

The apparatus 2 further comprises an electronic controller (not shown) which controls the supply of electrical power to the forced air flow device 70 and heating element 69. The electronic controller is connected to a thermistor 83 which is arranged to monitor the temperature of the heated base 82. Of course, the forced air flow device 70 and heating element 79 may be controlled by other suitable means. For example, the supply of power to the forced air flow device 70 and heating element 79 may instead be controlled by a thermomechanical arrangement similar to the control arrangement 80.

Operation of the apparatus 2 will now be described with reference to Figures 1-13 and also with reference to Figures 14 and 15 which illustrate air flow within the apparatus 2 during operation. A user may first pour water into the apparatus 2 for use in generating steam. This may be achieved by the user removing the measuring jug 34 and filling the measuring jug 34 with the required amount of water. The user may remove the manifold 14 and pour the water from the measuring jug 34 into the well 78 of the steam generator 40. The user may then replace the manifold 14 ready for operation of the apparatus 2. Alternatively, due to the openings 18 on the manifold 14, the user may leave the manifold 14 in the apparatus 2 and pour the water onto the manifold 14. The water will then drain through the openings 18 on the manifold 14 and collect in the well 78 on the steam generator 40. Once the user has filled the apparatus with the required amount of water, the user may then place the objects to be sterilised into the chamber 12. In the case of feeding equipment, this may involve placing an open bottle onto each of the nozzles 16 and placing any teats and covers onto the support structure 30. The user may then place the cover 6 onto the base 4 and press the power button 10 to begin a sterilisation and drying process.

Following pressing of the power button 10, the control arrangement 80 is configured to supply the sheathed heating element 84 with electrical power such that sheathed heating element 84 begins heating the base 82 thereby causing the water contained in the well 78 to be heated and produce steam. The steam produced by the steam generator 40 will rise and pass through the openings 18 and circulate around the chamber 12 and thus any objects placed therein. The steam acts to sterilise the objects in the chamber 12. This may be considered to be a sterilisation mode of operation. After a certain period of time has passed, or once all of the water has been evaporated to produce steam, the control arrangement 80 may cut the power to the heating element 84. For example, the control arrangement 80 may be configured to monitor a temperature of the heated base 82. Once the heated base 82 exceeds a pre-set threshold temperature, indicative of all of the water having evaporated, the control arrangement 80 may cut the power supply to the sheathed heating element 84.

The temperature of the heated base 82 is also monitored by the thermistor 83 which is connected to a suitable electronic controller. Once a threshold temperature, e.g. a temperature corresponding to all of the water having evaporated, is detected by the electronic controller, the electronic controller may supply the forced air flow device 70 and heating element 79 with power. This may be considered to be the initiation of a drying mode of operation. The forced air flow device 70 and heating element 79 may be arranged electrically in series such that whenever the forced air flow device 70 is operated, the heating element 79 is also operated.

Once the forced air flow device 70 is supplied with power, the forced air flow device 70 will draw air in through the air supply inlet 38 and force air, which is heated by the heating element 79, through the ducting 42 towards the manifold 14. With particular reference to Figure 6, the air which passes into the manifold 14 through the inlet portion 44 will be divided by the internal walls 50 and guided down each of the channels 52a-52f and further channels 54a-54d, and out through the nozzles 16 and air inlets 20 respectively.

With reference to Figure 14, air which passes out of the inlets 20 will be free to flow around the chamber 12. As discussed previously, the inlets 20 are arranged to direct at least a portion of air away from the chamber outlets 22. The air flow from the inlets 20 is illustrated by the arrows 86. The air flow for a single air inlet 20 is shown, but it will be appreciated that the air flow for the other air inlets 20 will be similar. As depicted, air which leaves the air inlet 20 travels in a first, upward, direction away from the chamber outlet 22. The air passes through the openings 32 in the support structure 30. As the air reaches the top of the chamber 12, it is redirected by the curved upper portion 7 of the cover 6 (not shown in this Figure), and eventually air flows within the chamber 12 cause the air to travel back towards the base of the chamber 12, whereby the air then passes out through the outlet 22. As depicted, this arrangement causes the air to circulate within the chamber 12, and thus the air may pass over the surfaces of objects within the chamber 12 and thereby dry the objects. The direction of air away from the chamber outlets 22, by the inlets 20, may help to prevent any air from bypassing the chamber 12 and passing directly out of the chamber outlets 22. Additionally, it may help to circulate air around the entire chamber 12 thus ensuring that all objects placed therein, irrespective of their position within the chamber 12, are dried by the air flow.

The air flow from the nozzles 16 will now be described with specific reference to Figure 15 which shows the air flow out of the nozzles 16 in the apparatus 2. The air flow for a single nozzle 16 is depicted and described, although it will be appreciated that the air flow out of each of the nozzles 16 may be the same. As depicted, air which passes out of the nozzle 16 has two distinct flows. The further outlet 24 which is aligned with an axis of the nozzle directs and creates an axial air flow 88 which is along the axis of the nozzle. Additionally, the outlets 26 form a helical air flow 90 which travels along and around the axis of the nozzle 16. When an object, e.g. an inverted open bottle, is placed onto the nozzle 16, the axial air flow 88 and helical air flow 90 will pass into and around the inside of the bottle. As discussed above, the Applicant has found the helical air flow 90 to be particularly efficient in drying the inside side walls of objects and the axial air flow 88 to be particularly well suited to drying the end of the objects, e.g. a base thereof. The combination of the helical air flow 90 and axial air flow 88 may thus efficiently and substantially dry the inside of objects placed on the nozzles 16. The air flow within the bottle will be reversed by the end of the bottle and air will escape the bottle through its opening and pass down through the plurality of openings 28 surrounding the nozzle 16. This air may then pass back up through the openings 28 into the chamber 12 where it may circulate and pass out through the outlets 22, shown in Figure 14.

With continued reference to Figures 1-15, and with particular reference to Figure 11, once the air has passed out through the chamber outlets 22, the air passes through the expansion chambers 74 and out through the apparatus outlets 36. As discussed above, the apparatus outlets 36 may have a larger cross-sectional area, i.e. have a larger opening, than the chamber outlets 22, and thus the air may pass out through the apparatus outlets 36 in a more diffuse manner. This may avoid a jet of heated air/steam passing out of the apparatus outlets 36.

The forced air flow device 70, and heating element 69, may operate for a fixed period of time, which may be controlled by the electronic controller. For example, after a fixed period of time has passed, the electronic controller may cut the power supply to the forced air flow device 70 and the heating element 79. The length of the fixed period of time may be dependent on certain characteristics of the apparatus, e.g. the size of the chamber, the number of objects within the chamber and/or the flow rate of air from the forced air flow device. Alternatively, the forced air flow device 70 and heating element 79 may be switched off once the temperature of the heated base, which may be measured by the thermistor 83, reaches a certain temperature.

The air flows generated by the nozzles 16 and air inlets 20 of the present invention may significantly reduce the drying time for objects within the apparatus 2. This may therefore reduce the time it takes to dry objects within the apparatus 2 ready for use. Once the air flow device 70 has stopped, the user may remove the cover 6 and remove the objects from within the chamber 12 which are sterilised and ready for use.

Whilst the forced air flow device 70 and heating element 79 may be arranged electrically in series, in alternative embodiments, the forced air flow device 70 and heating element 79 may be independently supplied with electrical power. This may allow the forced air flow device 70 and heating element 79 to be operated independently of one another. For example, the heating element 79 may be switched off prior to switching off of the forced air flow device. This may allow cooler air to be directed into the chamber 12, which may help to cool any objects within the apparatus 2 which may have become heated from the sterilisation process and the heated air flow from the forced air flow device 70 and heating element 79.

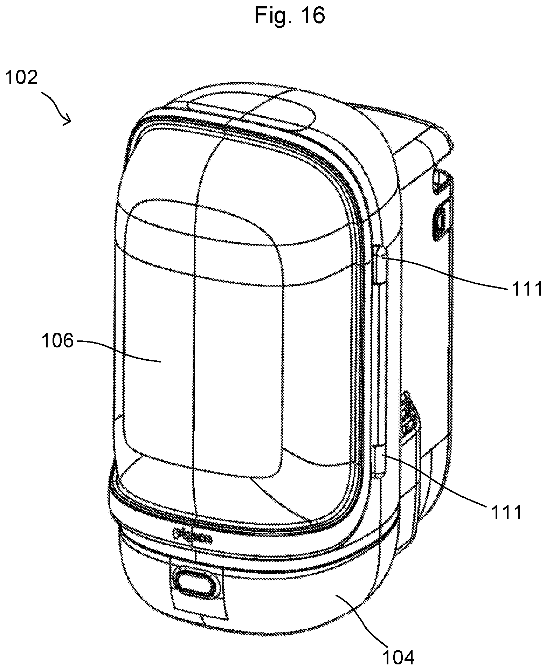

The apparatus 2 described above is just one exemplary apparatus in accordance with an embodiment of the present invention. Figure 16 shows a perspective view of another apparatus 102 for sterilising objects in accordance with another embodiment of the present invention. The apparatus 102 functions in a similar manner to the apparatus 2 described above. However, unlike the apparatus 2 which has a removable cover 6 which lifts away from the body 4, the apparatus 102 comprises a hinged door 106 which allows access to a chamber (not visible in this Figure) in which objects are contained for sterilisation. The door 106 may be made from a transparent or translucent material such that a user of the apparatus 102 can see into the chamber. The door 106 is connected to the body 104 by two hinges 111 which facilitate pivotal movement of the door 106 thereby allowing access to the chamber (not visible in this Figure).

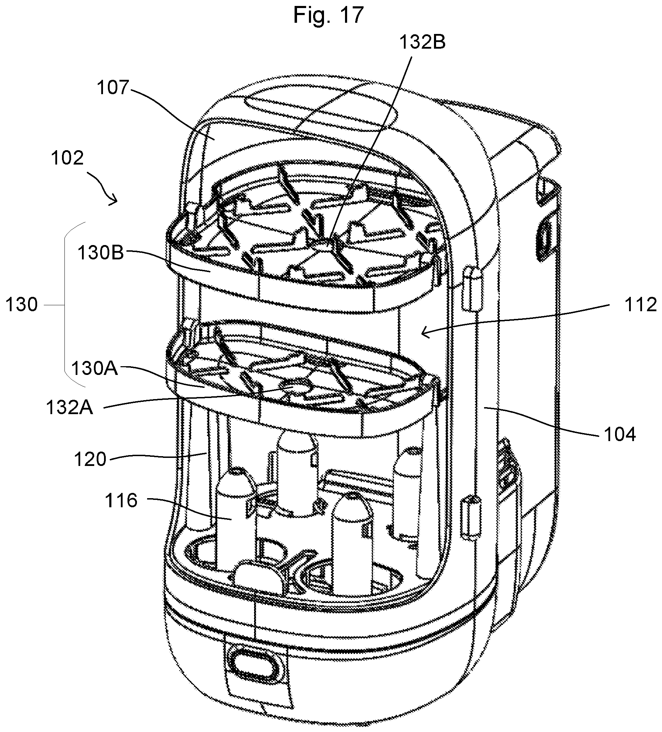

Figure 17 shows a perspective view of the apparatus 102 with the door 106 removed to reveal the chamber 112 and the components therein. As shown, similarly to the other embodiment described above, the apparatus 102 comprises nozzles 116 and air inlets 120 arranged at the base of the chamber 112. As can be seen, unlike the other embodiment which comprises six nozzles 16, and four air inlets 20, the apparatus 102 comprises four nozzles 116 and two air inlets 120. Thus, if the apparatus 102 is used for sterilising feeding equipment, the apparatus 102 may be used to sterilise four bottles and their associated components, with each bottle being placed onto one of the respective nozzles 116.

As shown, a support structure 130 is arranged in the chamber 112. In this embodiment, the support structure 130 comprises a first support structure 130A and a second support structure 130B. The first and second support structure 130A, 130B may be used to support items to be sterilised, e.g. teats or covers. The first and second support structures 130A, 130B comprise apertures 132A, 132B which allow the passage of steam and air therethrough during a sterilisation and drying process.

Similarly to the apparatus 2 described above, the apparatus 102 comprises an upper curved portion 107 which is arranged to redirect air within the chamber 112 back towards the base of the chamber 112. The upper curved portion 107 may be defined by both the body 104 and the hinged door 106 (not visible in this Figure).

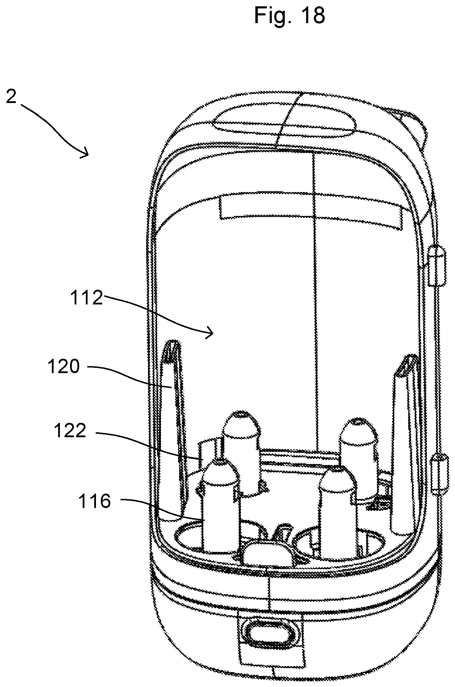

Figure 18 shows another view of the apparatus 102 with the support structure 130 removed to more clearly show the internal components of the apparatus 102. In this embodiment, the inlets 120 are in the form of conduits which extend towards the centre of the chamber 112 and above the nozzles 116. This may help to ensure that the air which leaves the inlets 120 is not disturbed by air leaving the nozzles 116 which do not have an object placed thereon. Figure 18 also more clearly shows the presence of a chamber outlet 122 at the rear of the chamber 112. Whilst one chamber outlet 122 can be seen, the apparatus 102 comprises another chamber outlet 122 arranged on the other side of the chamber 112. As shown, the inlets 120 are configured to direct air away from the outlet 122. By having the inlets 120 extend substantially into the chamber 112 in the manner shown in the form of conduits, this may further help to avoid any air leaving the inlets 120 and passing directly out of the outlet 122 and thus help to ensure the proper circulation of air within the chamber 112.

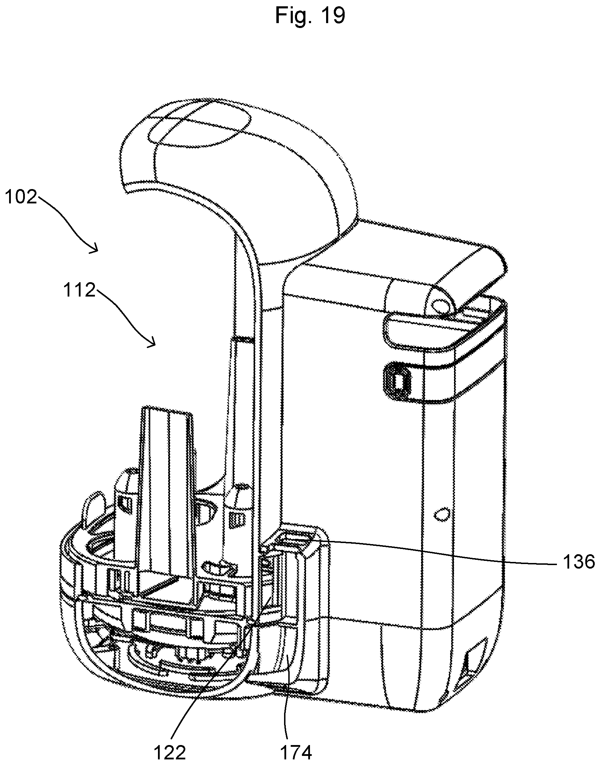

Figure 19 shows a cut-away view through the apparatus 102 to reveal the expansion chamber 174 arranged at the rear of the apparatus 112. As depicted, the chamber outlet 122 on the chamber 112 is in fluid communication with the expansion chamber 174. At the top of the expansion chamber 174 is an apparatus outlet 136 which is in fluid communication with the environment in which the apparatus 102 is placed. Accordingly, in a similar manner to the apparatus 2 described above, in use, steam and/or air may pass out of the chamber 112 into the expansion chamber 174 where it is then free to pass out through the further outlet 136. As the further outlet 136 may have a larger cross-sectional area than the chamber outlet 122, the expansion chamber 174 may reduce the pressure of the steam and/or air, and thus the steam and/or air may pass out through the further outlet 136 in a more diffuse manner. The expansion chamber 174 and apparatus outlet 136 direct steam and air vertically so as not to have the steam aimed horizontally at the user or nearby objects, for example those on a countertop.

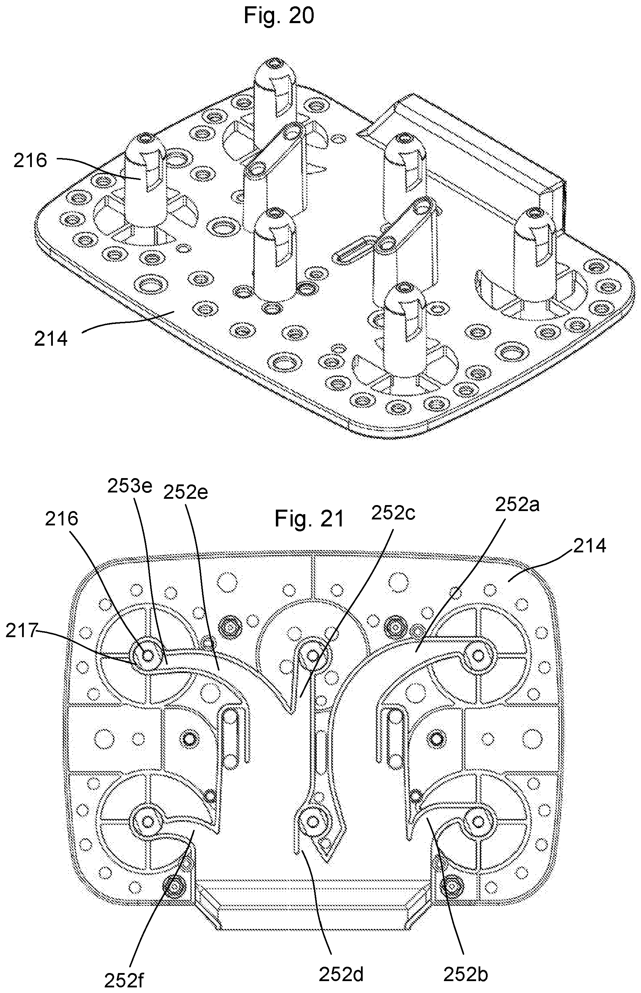

Figure 20 shows a perspective view of another manifold 214 which may be used in an apparatus according to the present invention. The manifold 214 is substantially identical to the manifold 14 described above, with the exception that the nozzles 216 (of which only one is labelled for clarity) do not comprise an internal helical air flow path as is the case for the nozzles 16 of the manifold 14 described above. Instead, air is directed into the nozzles 216 such that it is guided by an internal wall within the nozzle so as to flow in a helical manner.

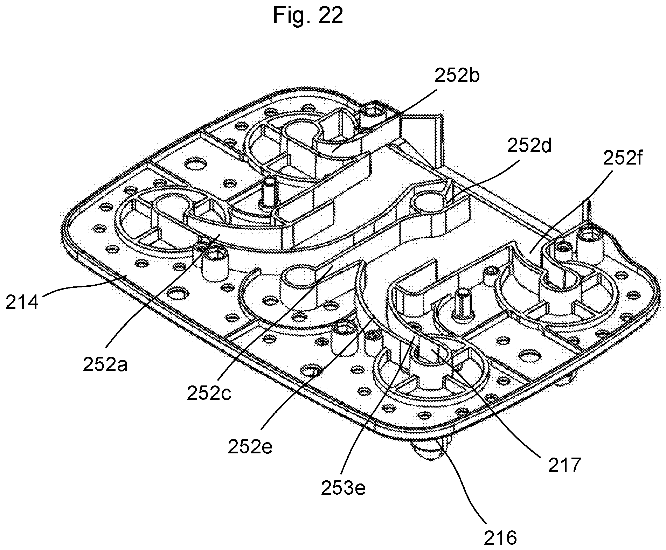

Figure 21 shows a plan view of the underside of the manifold 214 shown in Figure 20 and Figure 22 shows a perspective view of the underside of the manifold 214. With reference to the nozzle 216 in the top left of Figure 21, which corresponds to the nozzle 216 in the bottom right of Figure 22, the channel 252e comprises a channel outlet 253e which guides the air around an internal wall 217 of the nozzle 216. The nozzles 216 have a cylindrical shape and thus the internal wall 217 has a circular profile. Due to the circular profile of the internal wall 217, the air will flow in a helical manner within the nozzle 216. The Applicant has found that the air may form a helical air flow within the nozzle 216 even without the presence of a helical flow path as shown in earlier embodiments. Avoiding the need to provide a helical flow path within the nozzle 216 may simplify the manufacture of the apparatus, specifically the manifold 214 thereof. Whilst not described, it will be appreciated that each of the other channels 252a, 252b, 252c, 252d, 252f may comprise similar channel outlets which direct air into their respective nozzles such that air flows helically therein.

## Claims

1. An apparatus (2; 102), for sterilising objects, comprising:
a chamber (12; 112), for housing objects to be sterilised;
a sterilisation means (40) for sterilising objects within the chamber (12; 112);
a forced air flow device (70) arranged to direct air into the chamber (12; 112); and
at least one nozzle (16; 116; 216) extending into the chamber (12; 112) and arranged to direct air from the forced air flow device (70);
wherein the at least one nozzle (16; 116; 216) comprises at least one outlet (26) arranged to direct air along and around an axis of the nozzle (16; 116; 216) to thereby create a helical flow of air (90); and
**characterised in that**,
i) the nozzle (16; 116) comprises an internal helical air flow path (58) leading towards the outlet (26);
and/or
ii) air from the forced air flow device (70) is directed into the nozzle (16; 116; 216) so as to flow around an internal wall (17; 217) of the nozzle (16; 116; 216) and thereby create a helical flow of air within the nozzle (16; 116; 216).

2. An apparatus (2; 102) as claimed in claim 1, wherein the at least one nozzle (16; 116; 216) comprises a further outlet (24) aligned with an axis of the nozzle (16; 116; 216) and arranged to direct air along the axis of the nozzle (16; 116; 216).

3. An apparatus (2; 102) as claimed in any preceding claim, wherein the air enters the nozzle (16; 116; 216) at least partially tangentially with the internal wall (17; 217) of the nozzle (16; 116; 216).

4. An apparatus (2; 102) as claimed in any preceding claim, wherein the at least one outlet (26) comprises a plurality of outlets each arranged to direct air along and around the axis so as to create a helical flow of air (90); and/or wherein the at least one nozzle (16; 116; 216) comprises a plurality of nozzles.

5. An apparatus (2; 102) as claimed in any preceding claim, further comprising an air directing means (14; 214), located downstream of the forced air flow device (70), arranged to direct a first portion of air through the at least one nozzle (16; 116; 216) and a second portion of air directly into the chamber (12; 112); optionally wherein the first portion of air comprises between 40% and 60%, preferably 50%, of the air directed by the forced air flow device (70).

6. An apparatus (2; 102) of any preceding claim, wherein the sterilisation means (40) comprises a steam generator (40) arranged to direct steam into the chamber (12; 112).

7. An apparatus (2; 102) as claimed in any preceding claim, further comprising at least one inlet (20; 120) arranged to allow a portion of the air directed by the forced air flow (70) device into the chamber (12; 112), and at least one chamber outlet (22; 122) arranged to allow fluid to escape the chamber (12; 112), wherein the at least one inlet (20; 120) and the at least one chamber outlet (22; 122) are arranged in the chamber (12; 112) such that at least a portion of the air passing into the chamber (12; 112) through the at least one inlet (20; 120) travels in a first direction away from the at least one chamber outlet (22; 122).

8. An apparatus (2; 102) as claimed in claim 7, wherein the at least one chamber outlet (22; 122) is arranged on a lower portion of the chamber (12; 112).

9. An apparatus (2; 102) as claimed in claim 7 or 8, wherein the at least one inlet (20; 120) comprises a conduit (20; 120) which extends into the chamber (12; 112); optionally wherein the conduit (20; 120) extends from a base of the chamber (12; 112) towards a central portion of the chamber (12; 112).

10. An apparatus (2; 102) as claimed in any one of claims 7 to 9, wherein the at least one inlet (20; 120) is arranged above the at least one chamber outlet (22; 122); and/or wherein a portion (107) of the chamber (12; 112) facing the at least one air inlet (20; 120) is shaped so as to redirect air around the chamber (12; 112) towards the at least one air outlet (22; 122).

11. An apparatus (2; 102) as claimed in any one of claims 7 to 10, wherein a cross-sectional area of the at least one chamber outlet (22; 122) is less than a cross-sectional area of an air supply inlet (38) to the forced air flow device (70).

12. An apparatus (2; 102) as claimed in any one of claims 7 to 11, further comprising an expansion chamber (74; 174), arranged downstream of the at least one chamber outlet (22; 122), which comprises at least one apparatus outlet (36; 136) in fluid communication with the environment which the apparatus (2; 102) is arranged; optionally wherein the at least one apparatus outlet (36; 136) has a cross-sectional area which is larger than a cross-sectional area of the at least one chamber outlet (22; 122).

13. An apparatus (2; 102) as claimed in any preceding claim, wherein the forced air flow device (70) is arranged to direct air, from the environment in which the apparatus (2; 102) is placed, into the chamber (12; 112); and/or further comprising an electric heating element (79) arranged to heat the air directed by the forced air flow device (70).

14. An apparatus (2; 102) as claimed in any preceding claim, further comprising a one-way valve (71) arranged downstream of the forced air flow device (70) and configured to allow air to flow through the one-way valve (71) towards the chamber (12; 112); and/or wherein the apparatus (2; 102) is a baby equipment sterilising apparatus.

## Patentansprüche

1. Einrichtung (2; 102) zum Sterilisieren von Objekten, umfassend:
eine Kammer (12; 112) zum Aufnehmen von zu sterilisierenden Objekten;
ein Sterilisationsmittel (40) zum Sterilisieren von Objekten innerhalb der Kammer (12; 112);
eine Gebläsevorrichtung (70), die angeordnet ist, um Luft in die Kammer (12; 112) zu leiten; und
mindestens eine Düse (16; 116; 216), die sich in die Kammer (12; 112) erstreckt und angeordnet ist, um Luft aus der Gebläsevorrichtung (70) zu leiten;
wobei die mindestens eine Düse (16; 116; 216) mindestens einen Auslass (26) umfasst, der angeordnet ist, um Luft entlang und um eine Achse der Düse (16; 116; 216) herum zu leiten, um dadurch eine spiralförmige Luftströmung (90) zu schaffen; und
**dadurch gekennzeichnet, dass**
i) die Düse (16; 116) einen internen spiralförmigen Luftströmungsweg (58) umfasst, der in Richtung zum Auslass (26) führt; und/oder
ii) Luft aus der Gebläsevorrichtung (70) in die Düse (16; 116; 216) geleitet wird, um um eine Innenwand (17; 217) der Düse (16; 116; 216) herum zu strömen und dadurch eine spiralförmige Luftströmung innerhalb der Düse (16; 116; 216) zu schaffen.

2. Einrichtung (2; 102) nach Anspruch 1, wobei die mindestens eine Düse (16; 116; 216) einen weiteren Auslass (24) umfasst, der mit einer Achse der Düse (16; 116; 216) ausgerichtet und angeordnet ist, um Luft entlang der Achse der Düse (16; 116; 216) zu leiten.

3. Einrichtung (2; 102) nach einem vorstehenden Anspruch, wobei die Luft mindestens teilweise tangential zur Innenwand (17; 217) der Düse (16; 116; 216) in die Düse (16; 116; 216) eintritt.

4. Einrichtung (2; 102) nach einem vorstehenden Anspruch, wobei der mindestens eine Auslass (26) eine Vielzahl von Auslässen umfasst, die jeweils angeordnet sind, um Luft entlang und um die Achse herum zu leiten, um eine spiralförmige Luftströmung (90) zu schaffen; und/oder wobei die mindestens eine Düse (16; 116; 216) eine Vielzahl von Düsen umfasst.

5. Einrichtung (2; 102) nach einem vorstehenden Anspruch, weiter umfassend ein Luftleitmittel (14; 214), das sich stromabwärts von der Gebläsevorrichtung (70) befindet und angeordnet ist, um einen ersten Anteil der Luft durch die mindestens eine Düse (16; 116; 216) und einen zweiten Anteil der Luft direkt in die Kammer (12; 112) zu leiten; optional, wobei der erste Anteil der Luft zwischen 40 % und 60 %, vorzugsweise 50 %, der von der Gebläsevorrichtung (70) geleiteten Luft umfasst.

6. Einrichtung (2; 102) nach einem vorstehenden Anspruch, wobei das Sterilisationsmittel (40) einen Dampfgenerator (40) umfasst, der angeordnet ist, um Dampf in die Kammer (12; 112) zu leiten.

7. Einrichtung (2; 102) nach einem vorstehenden Anspruch, weiter umfassend mindestens einen Einlass (20; 120), der angeordnet ist, um einen Anteil der von der Gebläsevorrichtung (70) geleiteten Luft in die Kammer (12; 112) zu lassen, sowie mindestens einen Kammerauslass (22; 122), der angeordnet ist, um einem Fluid zu ermöglichen, aus der Kammer (12; 112) zu entweichen, wobei der mindestens eine Einlass (20; 120) und der mindestens eine Kammerauslass (22; 122) so in der Kammer (12; 112) angeordnet sind, dass mindestens ein Anteil der Luft, die durch den mindestens einen Einlass (20; 120) in die Kammer (12; 112) gelangt, sich in einer ersten Richtung weg von dem mindestens einen Kammerauslass (22; 122) bewegt.

8. Einrichtung (2; 102) nach Anspruch 7, wobei der mindestens eine Kammerauslass (22; 122) an einem unteren Abschnitt der Kammer (12; 112) angeordnet ist.

9. Einrichtung (2; 102) nach Anspruch 7 oder 8, wobei der mindestens eine Einlass (20; 120) eine Leitung (20; 120) umfasst, die sich in die Kammer (12; 112) erstreckt; optional, wobei sich die Leitung (20; 120) von einer Basis der Kammer (12; 112) in Richtung zu einem mittleren Abschnitt der Kammer (12; 112) erstreckt.

10. Einrichtung (2; 102) nach einem der Ansprüche 7 bis 9, wobei der mindestens eine Einlass (20; 120) über dem mindestens einen Kammerauslass (22; 122) angeordnet ist; und/oder wobei ein Abschnitt (107) der Kammer (12; 112), der dem mindestens einem Lufteinlass (20; 120) zugewandt ist, geformt ist, um Luft um die Kammer (12; 112) herum in Richtung zu dem mindestens einen Luftauslass (22; 122) umzuleiten.

11. Einrichtung (2; 102) nach einem der Ansprüche 7 bis 10, wobei eine Querschnittsfläche des mindestens einen Kammerauslasses (22; 122) kleiner ist als eine Querschnittsfläche eines Luftzufuhreinlasses (38) zu der Gebläsevorrichtung (70).

12. Einrichtung (2; 102) nach einem der Ansprüche 7 bis 11, weiter umfassend eine Expansionskammer (74; 174), die stromabwärts von dem mindestens einen Kammerauslass (22; 122) angeordnet ist und mindestens einen Einrichtungsauslass (36; 136) in Fluidverbindung mit der Umgebung, in der die Einrichtung (2; 102) angeordnet ist, umfasst; optional, wobei der mindestens eine Einrichtungsauslass (36; 136) eine Querschnittsfläche aufweist, die größer ist als die Querschnittsfläche des mindestens einen Kammerauslasses (22; 122).

13. Einrichtung (2; 102) nach einem vorstehenden Anspruch, wobei die Gebläsevorrichtung (70) angeordnet ist, um Luft aus der Umgebung, in der die Einrichtung (2; 102) platziert ist, in die Kammer (12; 112) zu leiten; und/oder weiter umfassend ein elektrisches Heizelement (79), das angeordnet ist, um die von der Gebläsevorrichtung (70) geleitete Luft zu erwärmen.

14. Einrichtung (2; 102) nach einem vorstehenden Anspruch, weiter umfassend ein Einwegventil (71), das stromabwärts von der Gebläsevorrichtung (70) angeordnet und dazu konfiguriert ist, zu ermöglichen, dass Luft durch das Einwegventil (71) in Richtung zu der Kammer (12; 112) strömen kann; und/oder wobei die Einrichtung (2; 102) eine Sterilisationseinrichtung für Babyausstattung ist.

## Revendications

1. Appareil (2 ; 102) pour stériliser des objets, comprenant :
une chambre (12 ; 112), pour loger des objets à stériliser ;
un moyen de stérilisation (40) pour stériliser des objets à l'intérieur de la chambre (12 ; 112) ;
un dispositif (70) de flux d'air forcé agencé pour guider l'air dans la chambre (12 ; 112) ; et
au moins une buse (16 ; 116 ; 216) s'étendant dans la chambre (12 ; 112) et agencée pour diriger l'air à partir du dispositif (70) de flux d'air forcé ;
dans lequel l'au moins une buse (16 ; 116 ; 216) comprend au moins une sortie (26) agencée pour diriger l'air le long et autour d'un axe de la buse (16 ; 116 ; 216) pour ainsi créer un flux d'air hélicoïdal (90) ; et
**caractérisé en ce que**,
i) la buse (16 ; 116) comprend un trajet interne (58) de flux d'air hélicoïdal menant vers la sortie (26) ; et/ou
ii) l'air provenant du dispositif (70) de flux d'air forcé est dirigé dans la buse (16 ; 116 ; 216) de manière à circuler autour d'une paroi interne (17 ; 217) de la buse (16 ; 116 ; 216) et à créer ainsi un flux d'air hélicoïdal à l'intérieur de la buse (16 ; 116 ; 216).

2. Appareil (2 ; 102) selon la revendication 1, dans lequel l'au moins une buse (16 ; 116 ; 216) comprend une autre sortie (24) alignée sur un axe de la buse (16 ; 116 ; 216) et agencée pour diriger l'air le long de l'axe de la buse (16 ; 116 ; 216).

3. Appareil (2 ; 102) selon une quelconque revendication précédente, dans lequel l'air pénètre dans la buse (16 ; 116 ; 216) de manière au moins partiellement tangentielle à la paroi interne (17 ; 217) de la buse (16 ; 116 ; 216).

4. Appareil (2 ; 102) selon une quelconque revendication précédente, dans lequel l'au moins une sortie (26) comprend une pluralité de sorties chacune agencées pour guider l'air le long et autour de l'axe de manière à créer un flux d'air hélicoïdal (90) ;
et/ou dans lequel l'au moins une buse (16 ; 116 ; 216) comprend une pluralité de buses.

5. Appareil (2 ; 102) selon une quelconque revendication précédente, comprenant en outre un moyen de guidage d'air (14 ; 214), situé en aval du dispositif (70) de flux d'air forcé, agencé pour guider une première partie d'air à travers l'au moins une buse (16 ; 116 ; 216) et une seconde partie d'air directement dans la chambre (12 ; 112) ; éventuellement, dans lequel la première partie d'air comprend entre 40 % et 60 %, de préférence 50 %, de l'air guidé par le dispositif (70) de flux d'air forcé.

6. Appareil (2 ; 102) selon une quelconque revendication précédente, dans lequel le moyen de stérilisation (40) comprend un générateur (40) de vapeur agencé pour guider de la vapeur dans la chambre (12 ; 112).

7. Appareil (2 ; 102) selon une quelconque revendication précédente, comprenant en outre au moins une entrée (20 ; 120) agencée pour permettre une partie de l'air guidé par le dispositif (70) de flux d'air forcé dans la chambre (12 ; 112), et au moins une sortie (22 ; 122) de chambre agencée pour permettre au fluide de s'échapper de la chambre (12 ; 112), dans lequel l'au moins une entrée (20 ; 120) et l'au moins une sortie (22 ; 122) de chambre sont agencées dans la chambre (12 ; 112) de sorte qu'au moins une partie de l'air passant dans la chambre (12 ; 112) à travers l'au moins une entrée (20 ; 120) circule dans une première direction en s'éloignant de l'au moins une sortie (22 ; 122) de chambre.

8. Appareil (2 ; 102) selon la revendication 7, dans lequel l'au moins une sortie (22 ; 122) de chambre est agencée sur une partie inférieure de la chambre (12 ; 112).

9. Appareil (2 ; 102) selon la revendication 7 ou la revendication 8, dans lequel l'au moins une entrée (20 ; 120) comprend un conduit (20 ; 120) qui s'étend dans la chambre (12 ; 112) ; éventuellement, dans lequel le conduit (20 ; 120) s'étend d'une base de la chambre (12 ; 112) vers une partie centrale de la chambre (12 ; 112).

10. Appareil (2 ; 102) selon l'une quelconque des revendications 7 à 9, dans lequel l'au moins une entrée (20 ; 120) est agencée au-dessus de l'au moins une sortie (22 ; 122) de chambre ; et/ou dans lequel une partie (107) de la chambre (12 ; 112) faisant face à l'au moins une entrée (20 ; 120) d'air est formée de manière à rediriger l'air autour de la chambre (12 ; 112) vers l'au moins une sortie (22 ; 122) d'air.

11. Appareil (2 ; 102) selon l'une quelconque des revendications 7 à 10, dans lequel une surface de section transversale de l'au moins une sortie (22 ; 122) de chambre est inférieure à une surface de section transversale d'une entrée (38) d'alimentation en air vers le dispositif (70) de flux d'air forcé.

12. Appareil (2 ; 102) selon l'une quelconque des revendications 7 à 11, comprenant en outre une chambre (74 ; 174) d'expansion, agencée en aval de l'au moins une sortie (22 ; 122) de chambre, qui comprend au moins une sortie (36 ; 136) d'appareil en communication fluidique avec l'environnement dans lequel est agencé l'appareil (2 ; 102) ; éventuellement dans lequel l'au moins une sortie (36 ; 136) d'appareil présente une surface de section transversale qui est plus grande qu'une surface de section transversale de l'au moins une sortie (22 ; 122) de chambre.

13. Appareil (2 ; 102) selon une quelconque revendication précédente, dans lequel le dispositif (70) de flux d'air forcé est agencé pour guider l'air, à partir de l'environnement dans lequel est placé l'appareil (2 ; 102), dans la chambre (12 ; 112) ; et/ou comprenant en outre un élément chauffant électrique (79) agencé pour chauffer l'air guidé par le dispositif (70) de flux d'air forcé.

14. Appareil (2 ; 102) selon une quelconque revendication précédente, comprenant en outre une vanne unidirectionnelle (71) agencée en aval du dispositif (70) de flux d'air forcé et configurée pour permettre à l'air de passer par la vanne unidirectionnelle (71) vers la chambre (12 ; 112) ; et/ou dans lequel l'appareil (2 ; 102) est un appareil de stérilisation d'équipement pour bébé.
